# EUROPEAN PATENT SPECIFICATION

(11) **EP 1 388 538 B1**
(45) Date of publication and mention of the grant of the patent: **01.09.2010**
(21) Application number: 03014990.0
(22) Date of filing: 02.07.2003
(51) Int. Cl.: C07D 295/10, G03F 7/031, C09K 19/54, G02F 1/1333

(54) **Polymerisation Initiator**
Polymerizationsinitiator
Initiateur de polymérisation

(30) Priority: 09.07.2002 EP 02015208
(43) Date of publication of application: 11.02.2004
(73) Proprietor: Merck Patent GmbH, 64293 Darmstadt (DE)
(72) Inventor: Eastwood, Amanda, St Denys Southampton HANTS SO17 2NU (GB); Parri, Owain Llyr, Poole, Dorset BH15 2LN (GB); Slaney, Kim, Christchurch, Dorset BH23 1QX (GB)

(56) References cited:
- GB-A- 2 320 027
- GB-A- 2 355 720
- US-A- 4 559 371
- US-A- 4 582 862
- US-A- 5 801 212
- US-A- 6 074 708
- DATABASE WPI Section Ch, Week 199006 Derwent Publications Ltd., London, GB; Class A26, AN 1990-039838 XP002256551 & JP 01 315423 A (IDEMITSU KOSAN CO.), 20 December 1989 (1989-12-20)
- RUTSCH, W. ET AL: "New photoinitiators for pigmented systems" ORGANIC COATINGS (1986), 8, 175-95, 1986, XP008021965
- CROMWELL N. H. ET AL.: "Elimination reaction of alpha-halogenated ketones. IV. Elimination-substitution reactions with alpha-bromo-p-phenylisobutyrophenone" JOURNAL OF THE AMERICAN CHEMICAL SOCIETY., vol. 83, no. 5, 1961, pages 1237-1240, XP002256550 AMERICAN CHEMICAL SOCIETY, WASHINGTON, DC., US ISSN: 0002-7863
- "Flüssige Kristalle" Römpp Online, Version 3.2 [retrieved on 2008-08-26]
- DIRK J. BOER ET AL.: "Photo-Induced Diffusion in Polymerizing Chiral-Nematic Media" ADVANCED MATERIALS, vol. 11, no. 7, 1999, pages 573-578, XP002237446

## Description

### Field of the Invention

The invention relates to compounds that can be used as photoinitiators for polymerization, to their synthesis, to polymerizable compositions and polymers obtained with these compounds, and to the use of the compounds, compositions and polymers in optical, electrooptical, semiconductor and photoconductor applications.

### Background and Prior Art

Photochemically induced polymerization reactions have attained great importance in industry, particularly in the rapid hardening of thin films, as, for example, in the hardening of paint or resin coatings on paper, metal and plastics or in the drying of printing inks, since these processes are distinguished from conventional methods for printing and coating articles by raw material and energy savings and reduced environmental pollution. However, the preparation of polymeric materials by polymerizing appropriate unsaturated monomeric starting materials is frequently effected photochemically, for which customary processes such as solution and emulsion polymerizations can be used.

Since with the aforementioned reactions it is generally the case that none of the reactants is capable of absorbing photochemically active radiation to an adequate degree, it is necessary to add so-called photoinitiators which are either capable of absorbing incident high-energy radiation, usually UV light, and of forming active starter radicals which in turn initiate photopolymerization, or are capable of transferring the absorbed energy for free radical formation to one of the polymerizable reactants. The initiators do not normally participate in the actual polymerization reaction.

The initiators used hitherto for photopolymerizing unsaturated compounds have been in the main benzophenone derivatives, benzoin ethers, benzil ketals, dibenzosuberone derivatives, anthraquinones, xanthenones, thioxanthones, .alpha.-halogenactophenone derivatives, dialkoxyacetophenones and hydroxyalkyl phenones.

Essential criteria for the selection of such initiators are, for example, the nature of the reactions to be carried out, the ratio of the absorption spectrum of the initiator to the spectral energy distribution of the available source of irradiation, the activity of the initiator, the solubility of the initiator in the reaction mixture, the dark storage life of the reaction system to which the initiator has been added, and the action on the end products by residues remaining therein of the initiator and/or of the products formed therefrom in the course of the photochemical reaction.

As is known, however, the technical feasibility of many of the substances mentioned is restricted, in some instances severely, by a number of disadvantages. These particularly include insufficient reactivity to initiate the photopolymerization of ethylenically unsaturated compounds. In addition to molecule-specific reactivity, the crucial factor here in many cases is the solubility, or the ideally uniform incorporability of the photoinitiators in the photopolymerizable systems.

One important field of use of photoinitators is the preparation of liquid crystal (LC) polymers and LC polymer gels from polymerizable LC materials by photopolymerization. LC polymers are suitable for example in the preparation of anisotropic polymer films with uniform orientation, which found widespread use for example as optical films in displays. LC polymer gels are suitable for example as switchable components of an LC display device like for example a PDLC (polymer dispersed liquid crystal) or polymer gel type display. Other uses of polymerizable LC materials are for example the preparation of LC pigments, or the preparation of coloured or patterned LC films for decorative or security applications.

For the above uses the polymerizable LC material is typically coated onto a substrate, or provided between two substrates, oriented in its mesophase and polymerized by exposure to heat or photoradiation in the presence of a photinitiator absorbing at the wavelength of the photoradiation. For example, when polymerizing by means of UV light, a photoinitiator can be used that decomposes under UV irradiation to produce free radicals or ions that start the polymerization reaction. It is also possible to use a polymerization initiator that decomposes when heated to produce free radicals or ions that start the polymerization. Typical photoinitiators for radical polymerization are for example the commercially available Irgacure® or Darocure® initiators (from Ciba Geigy AG).

JP-A-10-069079, JP-A-10-090890 and JP-A-10-090891 disclose a photopolymerizable composition that can be used for the preparation of colour filters for LC displays and comprises a photoinitiator of the formula

Ar₁-CO-CR¹R²X

wherein Ar₁ is e.g. a phenyl group that is optionally substituted, or a phenylene-T-phenylene group wherein T is O, S or CH₂, R¹ and R² are an alkyl or phenylalkyl group, and X is a morpholine, amino, alkoxy or siloxane group.

US 6,074,708 discloses a photoinitiator having a structure as shown below for use in a photopolymerizable resin that can be polymerized to form a polymer wall in the switchable cell of an LC display device:

CₙH₂ₙ₊₁-(A)ₚ-(B)_{q}-phenylene-CO-C(OX)YZ

wherein A and B are e.g. cyclohexylene or phenylene, n is 1-9, p and q are 0 or 1, X is H or C₁₋₃-alkyl, Y is C₁₋₂-alkyl or C₁₋₃-alkoxy and Z is C₁₋₂-alkyl, alkylated phenyl or -phenylene-(B)_{q}-(A)ₚ-CₙH₂ₙ₊₁.

However, the photoinitiators as disclosed in JP-A-10-069079, JP-A-10-090890, JP-A-10-090891 and US 6,074,708 having an oxygen atom in α-position to the cleavage site require high cleavage energy, since the adjacent carbon radical is destabilized by the oxygen atom, which prevents fast curing.

Also, for polymerization of LC material the commercially available initiators like Irgacure are disadvantageous, as they are small molecules which do not have the shape of a typical liquid crystal molecule and therefore disrupt the packing and alignment of the LC material.

It was also suggested in prior art to use photoinitiators having a rod like molecular structure or showing liquid crystal behaviour. Broer et al., Adv. Mater. 1999, 11(7), 573-578 disclose a liquid crystalline photoinitiator of the formula 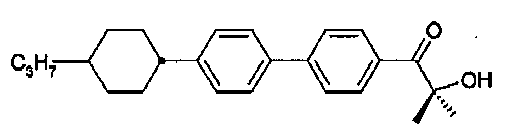 for the preparation of a cholesteric LC network. However, the initiator reported by Broer et al. is often too weak to offer the possibility of polymerizing an LC material at an open surface (i.e. the material being coated onto a substrate but not being covered with a second substrate), and requires photopolymerization with high power of radiation and in the absence of oxygen.

GB -A-2 320 027, JP 01-315423 A, US-A-5 801 212, US-A-4 559 371, US-A-4 582 862, GB-A-2 355 720, Rutsch et al., Org. Coat. 8, 175 (1986), and Cromwell et al., J. Am. Chem. Soc. 83(5), 1237 (1961) disclose various photocleavable or photodegradable compounds, some of which may also be used as photoinitiators, but do not disclose compounds as claimed in the present invention.

It was an aim of the present invention to provide compounds that can be used as polymerization initiators and do not have the drawbacks of prior art mentioned above, in particular photoinitiators that allow fast curing with reduced energy and, if used in polymerizable LC materials, do not disturb the packing and alignment of the LC material and do not negatively affect the LC phase behaviour of the LC material. Preferably these photoinititators should have a mesogenic or liquid crystalline, in particular a rod-like structure, and in the ideal case show liquid crystalline phase behaviour.

Further aims of this invention relate to a polymerizable LC composition comprising an initiator according to this invention, which is particularly suitable for the preparation of LC polymers and oriented LC polymer films, and to LC polymer and polymer films obtained from the initiators and compositions according to this invention.

A further aim of this invention is to provide an advantageous use of an initiator, composition or polymer according to this invention.

Further aims of this invention relate to optical, electrooptical, semiconductor and photoconductor devices, and to decorative or security markings or and optical data storage devices comprising an initiator, composition or polymer according to this invention.

Other aims of the present invention are immediately evident to the person skilled in the art from the following detailed description.

The inventors have found that these aims can be achieved by providing compounds as described in claim 1.

### Summary of the Invention

One aspect of the invention is a compound of formula 14 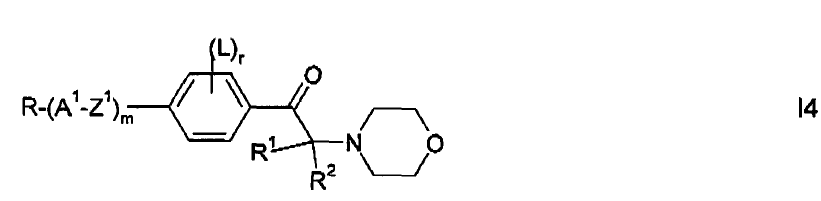 wherein
- R: is or straight chain or branched alkyl with 1 to 20 C-atoms, which may be unsubstituted, mono- or poly-substituted by F, Cl, Br, I or CN, it being also possible for one or more non-adjacent CH₂ groups to be replaced, in each case independently from one another, by -O-, -S-, -NH-, -NR⁰-, -SiR⁰R⁰⁰-, -CO-, -COO-, -OCO-, -OCO-O-, -S- CO-, -CO-S-, -CH=CH- or -C≡C- in such a manner that O and/or S atoms are not linked directly to one another,
- R⁰ and R⁰⁰: are independently of each other H or alkyl with 1 to 4 C atoms,
- R¹ and R²: are methyl or ethyl, wherein - CR¹R²- is different from -C(CH₃)(C₂H₅)-,
- A₁: is 1,4-phenylene, which is unsubstituted, or mono- or polysubstituted by L,
- L: is F or is 0, 1, 2, 3, or 4,
- Z¹: is -C≡C-, or a single bond, and
- m: is 2, 3 or 4

Another aspect of the present invention is a polymerizable composition, in particular a polymerizable liquid crystal composition, comprising at least one compound of formula 14 and at least one polymerizable compound.

Another aspect of the present invention is a polymer or polymer film obtained by polymerizing one or more polymerizable compounds in the presence of at least one compound of formula 14.

Another aspect of the present invention is an anisotropic polymer or polymer film obtained by polymerizing or copolymerizing a polymerizable liquid crystal composition comprising at least one compound of formula 14, wherein said composition is oriented in its liquid crystal phase.

Another aspect of the present invention is the use of compounds of formula 14, polymerizable compositions and polymers prepared thereof in optical films, polarizers, compensators, beam splitters, reflective films, alignment layers, colour filters, holographic elements, hot stamping foils, coloured images, decorative or security markings, liquid crystal pigments, adhesives, synthetic resins with anisotropic mechanical properties, cosmetics, diagnostics, nonlinear optics, optical information storage, as chiral dopants, in electronic devices like for example field effect transistors (FET) as components of integrated circuitry, as thin film transistors in flat panel display applications or for Radio Frequency Identification (RFID) tags, or in semiconducting components for organic light emitting diode (OLED) applications such as electroluminescent displays or backlights of e.g. liquid crystal displays, for photovoltaic or sensor devices, as electrode materials in batteries, as photoconductors, or for electrophotographic applications like electrophotographic recording.

Another aspect of the present invention is a liquid crystal display comprising in its active layer a polymerizable composition comprising at least one compound of formula 14, or comprising in its active layer a polymer obtained by polymerizing or copolymerizing one or more polymerizable compounds in the presence of at least one compound of formula 14.

### Definition of Terms

The term 'film' as used in this application includes self-supporting, i.e. free-standing, films that show more or less pronounced mechanical stability and flexibility, as well as coatings or layers on a supporting substrate or between two substrates.

The term 'liquid crystal or mesogenic material' or 'liquid crystal or mesogenic compound' means materials or compounds comprising one or more rod-shaped, board-shaped or disk-shaped mesogenic groups, i.e. groups with the ability to induce liquid crystal phase behaviour. Liquid crystal compounds with rod-shaped or board-shaped groups are also known in the art as 'calamitic' liquid crystals. Liquid crystal compounds with a disk-shaped group are also known in the art as 'discotic' liquid crystals. The compounds or materials comprising mesogenic groups do not necessarily have to exhibit a liquid crystal phase themselves. It is also possible that they show liquid crystal phase behaviour only in mixtures with other compounds, or when the mesogenic compounds or materials, or the mixtures thereof, are polymerized.

For the sake of simplicity, the term 'liquid crystal material' is used hereinafter for both liquid crystal materials and mesogenic materials, and the term 'mesogen' is used for the mesogenic groups of the material.

### Detailed Description of the Invention

The compounds of formula 14 include a stabilising N atom in α-position to the cleavage site. This stabilises the adjacent carbon radical to a greater degree than for example oxygen, therefore less energy is required for cleavage, promoting faster polymerization.

The compounds of formula 14 have a rod-like structure. Therefore, when used together with LC compounds or in an LC mixture, they do not disrupt the packing and the alignment of the LC molecules of the host material, even if they do not exhibit liquid crystal phases themselves. They are therefore particularly suitable as photoinitiators for the photopolymerization of liquid crystal materials.

Preferred subformulae for the group -(A¹-Z¹)ₘ- are listed below. For reasons of simplicity, Phe in these groups is 1,4-phenylene, Phe L is a 1,4-phenylene group which is substituted by 1 to 4 groups L as defined above. The following list of preferred groups is also comprising their mirror images.

-Phe-Z-Phe-Z- II-3

-PheL-Z-Phe-Z- II-6

-PheL-Z-PheL-Z- II-8

-Phe-Z-Phe-Z-Phe-Z- II-9

-Phe-Z-Phe-Z-PheL-Z- II-15

-Phe-Z-PheL-Z-Phe-Z- II-16

-PheL-Z-Phe-Z-Phe-Z- II-17

-PheL-Z-Phe-Z-PheL-Z- II-18

-PheL-Z-PheL-Z-Phe-Z- II-19

-PheL-Z-PheL-Z-PheL-Z- II-20

Particularly preferred is the subformula II-3

In these preferred groups Z in each case independently has one of the meanings of Z¹ as given in formula I. .

Very preferably the group -(A¹-Z¹)ₘ- is selected from the following formulae and their mirror images 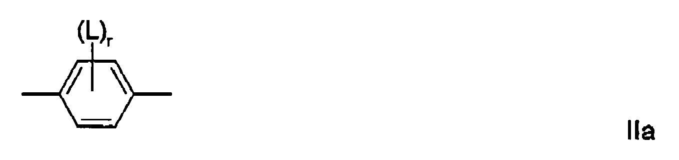 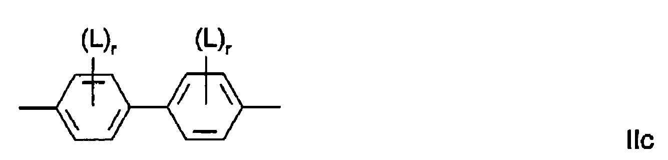 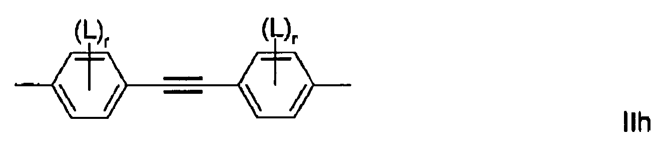 wherein L has the meaning given above and r is 0, 1 or 2.

The group 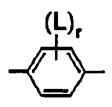 in these preferred formulae is very preferably denoting 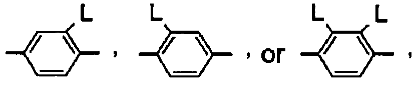 furthermore 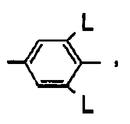 with L having each independently one of the meanings given above.

Especially preferred are compounds of formula 14 comprising at least one group 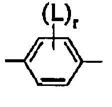 wherein r is 1.

Further preferred are compounds of formula 14 comprising at least two groups 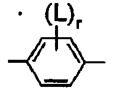 wherein r is 1 and/or at least one group 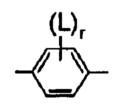 wherein r is 2.

The group Phe in formula 14 is preferably unsubstituted 1.4-phenylene. furthermore 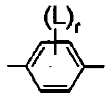 as defined above.

Especially preferred are compounds of formula I and the preferred formulae shown above and below wherein R is different from H.

Especially preferred are compounds of formula 14 and the preferred formulae shown above and below wherein R is selected from an alkyl, alkoxy or alkenyl radical.

If R and R¹⁻⁵² in formula 14 are an alkyl or alkoxy radical, i.e. where the terminal CH₂ group is replaced by -O-, this may be straight-chain or branched. It is preferably straight-chain, has 2, 3, 4, 5, 6, 7 or 8 carbon atoms and accordingly is preferably ethyl, propyl, butyl, pentyl, hexyl, heptyl, octyl, ethoxy, propoxy, butoxy, pentoxy, hexoxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Oxaalkyl, i.e. where one CH₂ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

If R in formula 14 is an alkyl group wherein one or more CH₂ groups are replaced by -CH=CH-, this may be straight-chain or branched. It is preferably straight-chain, has 2 to 10 C atoms and accordingly is preferably vinyl, prop-1-, or prop-2-enyl, but-1-, 2- or but-3-enyl, pent-1-, 2-, 3- or pent-4-enyl, hex-1-, 2-, 3-, 4- or hex-5-enyl, hept-1-, 2-, 3-, 4-, 5- or hept-6-enyl, oct-1-, 2-, 3-, 4-, 5-, 6- or oct-7-enyl, non-1-, 2-, 3-, 4-, 5-, 6-, 7- or non-8-enyl, dec-1-, 2-, 3-, 4-, 5-, 6-, 7-, 8- or dec-9-enyl.

Especially preferred alkenyl groups are C₂-C₇-1E-alkenyl, C₄-C₇-3E-alkenyl, C₅-C₇-4-alkenyl, C₆-C₇-5-alkenyl and C₇-6-alkenyl, in particular C₂-C₇-1E-alkenyl, C₄-C₇-3E-alkenyl and C₅-C₇-4-alkenyl. Examples for particularly preferred alkenyl groups are vinyl, 1E-propenyl, 1E-butenyl, 1E-pentenyl, 1E-hexenyl, 1E-heptenyl, 3-butenyl, 3E-pentenyl, 3E-hexenyl, 3E-heptenyl, 4-pentenyl, 4Z-hexenyl, 4E-hexenyl, 4Z-heptenyl, 5-hexenyl, 6-heptenyl and the like. Groups having up to 5 C atoms are generally preferred.

If R in formula 14 is an alkyl group, wherein one CH₂ group is replaced by -O- and one by -CO-, these radicals are preferably neighboured. Accordingly these radicals together form a carbonyloxy group -CO-O- or an oxycarbonyl group -O-CO-. Preferably this group R is straight-chain and has 2 to 6 C atoms.

It is accordingly preferably acetyloxy, propionyloxy, butyryloxy, pentanoyloxy, hexanoyloxy, acetyloxymethyl, propionyloxymethyl, butyryloxymethyl, pentanoyloxymethyl, 2-acetyloxyethyl, 2-propionyloxyethyl, 2-butyryloxyethyl, 3-acetyloxypropyl, 3-propionyloxypropyl, 4-acetyloxybutyl, methoxycarbonyl, ethoxycarbonyl, propoxycarbonyl, butoxycarbonyl, pentoxycarbonyl, methoxycarbonylmethyl, ethoxycarbonylmethyl, propoxycarbonylmethyl, butoxycarbonylmethyl, 2-(methoxycarbonyl)ethyl, 2-(ethoxycarbonyl)ethyl, 2-(propoxycarbonyl)ethyl, 3-(methoxycarbonyl)propyl, 3-(ethoxycarbonyl)propyl, 4-(methoxycarbonyl)-butyl.

If R in formula 14 is an alkyl group, wherein two or more CH₂ groups are replaced by -O- and/or -COO-, it can be straight-chain or branched. It is preferably straight-chain and has 3 to 12 C atoms. Accordingly it is preferably bis-carboxy-methyl, 2,2-bis-carboxy-ethyl, 3,3-bis-carboxy-propyl, 4,4-bis-carboxy-butyl, 5,5-bis-carboxy-pentyl, 6,6-bis-carboxy-hexyl, 7,7-bis-carboxy-heptyl, 8,8-bis-carboxy-octyl, 9,9-bis-carboxy-nonyl, 10,10-bis-carboxy-decyl, bis-(methoxycarbonyl)-methyl, 2,2-bis-(methoxycarbonyl)-ethyl, 3,3-bis-(methoxycarbonyl)-propyl, 4,4-bis-(methoxycarbonyl)-butyl, 5,5-bis-(methoxycarbonyl)-pentyl, 6,6-bis-(methoxycarbonyl)-hexyl, 7,7-bis-(methoxycarbonyl)-heptyl, 8,8-bis-(methoxycarbonyl)-octyl, bis-(ethoxycarbonyl)-methyl, 2,2-bis-(ethoxycarbonyl)-ethyl, 3,3-bis-(ethoxycarbonyl)-propyl, 4,4-bis-(ethoxycarbonyl)-butyl, 5,5-bis-(ethoxycarbonyl)-hexyl.

If R in formula 14 is an alkyl or alkenyl group that is monosubstituted by CN or CF₃, it is preferably straight-chain. The substitution by CN or CF₃ can be in any desired position.

If R in formula 14 is an alkyl or alkenyl group that is at least monosubstituted by halogen, it is preferably straight-chain. Halogen is preferably F or Cl, in case of multiple substitution preferably F. The resulting groups include also perfluorinated groups. In case of monosubstitution the F or Cl substituent can be in any desired position, but is preferably in ω-position. Examples for especially preferred straight-chain groups with a terminal F substituent are fluormethyl, 2-fluorethyl, 3-fluorpropyl, 4-fluorbutyl, 5-fluorpentyl, 6-fluorhexyl and 7-fluorheptyl. Other positions of F are, however, not excluded.

Halogen is preferably F or Cl.

Especially preferred are the following compounds 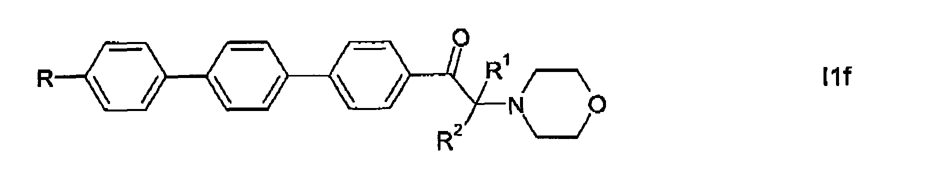 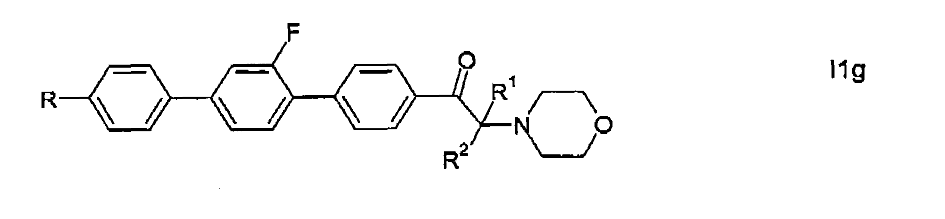 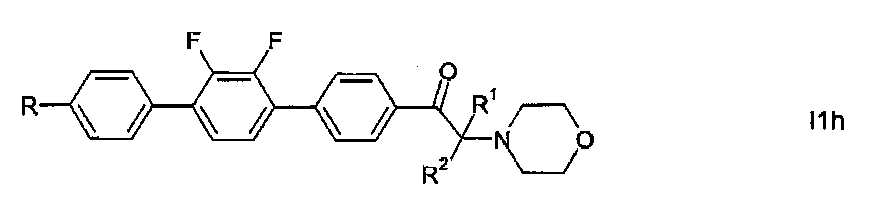 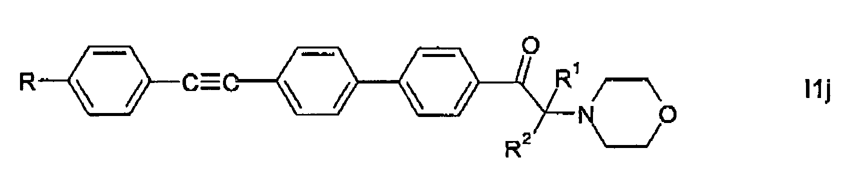 wherein R and R¹⁻² have the meanings given above, R is preferably alkyl or alkoxy with 1 to 8 C-atoms, R¹ and R² are alkyl with 1 or 2 C-atoms.

The compounds of formula 14 can be synthesized according to or in analogy to methods which are known per se and which are described in standard works of organic chemistry such as, for example, Houben-Weyl, Methoden der organischen Chemie, Thieme-Verlag, Stuttgart. Some specific methods of preparation can be taken from the examples.

The initiators of formula 14 can be used in a polymerizable composition, in particular a polymerizable LC composition, comprising at least one compound of formula 14 and at least one polymerizable compound.

They are further suitable for the preparation of polymers or polymer films by polymerizing or copolymerizing a polymerizable liquid crystal composition comprising at least one compound of formula 14, or by polymerizing one or more polymerizable compounds in the presence of at least one initiator of formula 14.

A polymerizable material according to the present invention preferably comprises 0.01 to 10 %, very preferably 0.05 to 5 %, in particular 0.1 to 3 % of an initiator of formula 14.

The polymerizable compositions comprising one or more initiators of formula 14 are particularly suitable for use in displays, in particular LC displays that comprise a polymer or polymer network component for the purpose of assisting alignment, mesophase stability and/or electrooptical property improvement, in particular to achieve faster response times and/or lower threshold voltages, or for the purpose to create a multidomain structure to achieve improved contrast at wide viewing angles. Such displays are for example of the TN, STN, ECB, VA, IPS, multidomain or hybrid mode, and are described for example in US 5,189,540, US 6,177,972, EP 0 903 392, and Hasebe et al., Jpn.J.Appl.Phys.1994, 33, 6245.

The compounds of formula 14 are also suitable for polymerizable compositions or polymer gels used in polymer stabilised displays, such as bistable PSCT (polymer stabilized cholesteric texture) displays, or PDLC or polymer gel displays of the scattering type. Anisotropic polymer gels and displays comprising them are disclosed for example in DE 195 04 224, GB 2 279 659, WO 93/22397, US 5,538,768, US 5,543,075 and EP 0 451 905

The compounds of formula 14 and polymerizable LC compositions comprising them are further useful for the preparation of anisotropic polymer films or coatings that can be used for example as optical films in displays or other optical devices.

A polymerizable LC composition according to the invention preferably comprises at least one polymerizable mesogenic compound having one polymerizable functional group (monoreactive compound) and at least one polymerizable mesogenic compound having two or more polymerizable functional groups (di- or multireactive compound).

If di- or multireactive compounds are present in the polymerizable material, a three-dimensional polymer network is formed and the planar orientation of the LC material is permanently fixed. A polymer film made of such a network is self-supporting and shows a high mechanical and thermal stability and a low temperature dependence of its physical and optical properties.

By varying the concentration of the di- and multireactive compounds the crosslink density of the polymer film and thereby its physical and chemical properties such as the glass transition temperature, which is also important for the temperature dependence of the optical properties of the optical retardation film, the thermal and mechanical stability or the solvent resistance can be tuned easily.

The polymerizable mesogenic mono-, di- or multireactive compounds used for the present invention can be prepared by methods which are known per se and which are described, for example, in standard works of organic chemistry such as, for example, Houben-Weyl, Methoden der organischen Chemie, Thieme-Verlag, Stuttgart. Typical examples are described for example in WO 93/22397; EP 0 261 712; DE 19504224; DE 4408171 and DE 4405316. The compounds disclosed in these documents, however, are to be regarded merely as examples that do not limit the scope of this invention.

Examples representing especially useful mono- and direactive polymerizable mesogenic compounds are shown in the following list of compounds, which should, however, be taken only as illustrative and is in no way intended to restrict, but instead to explain the present invention:

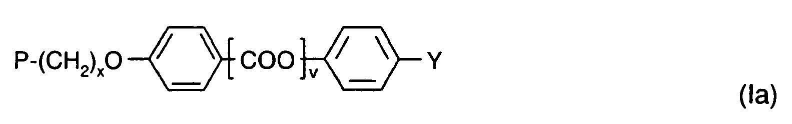

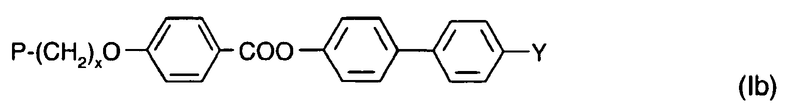

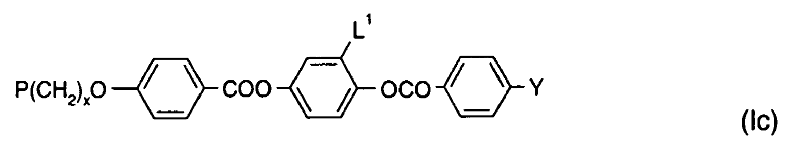

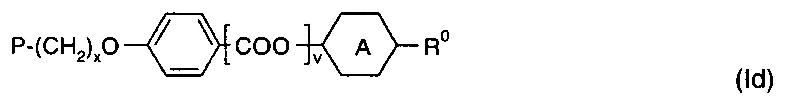

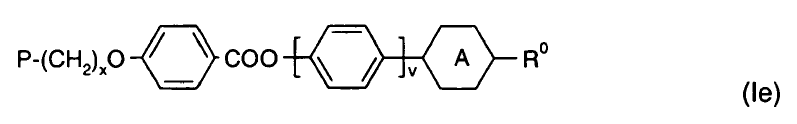

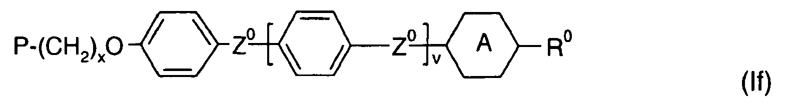

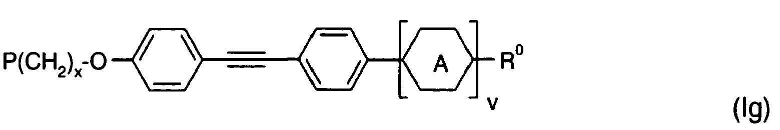

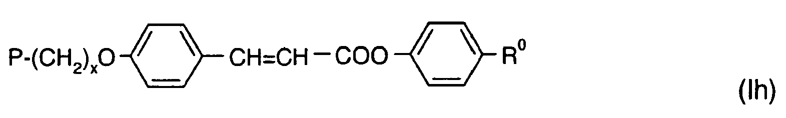

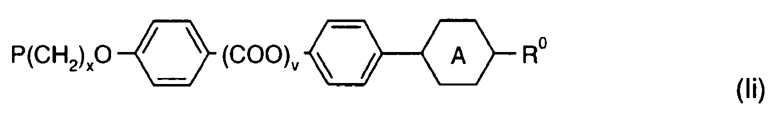

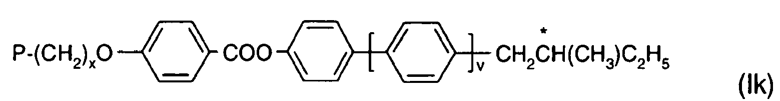

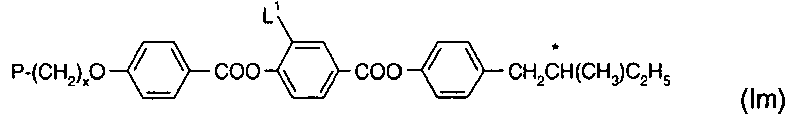

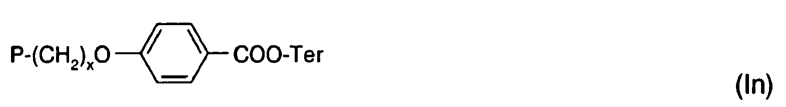

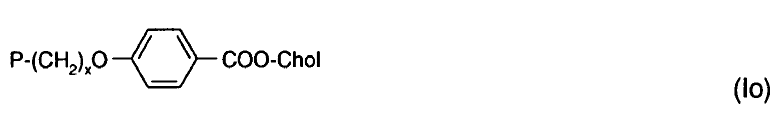

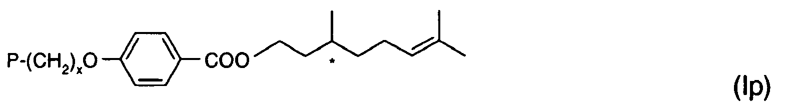

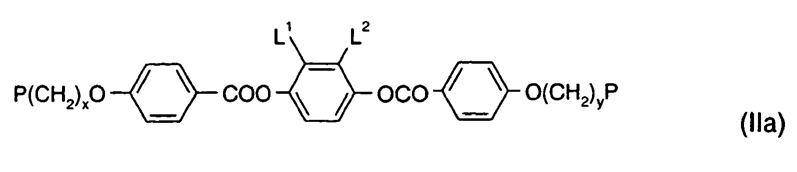

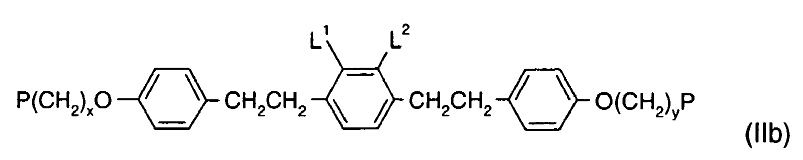

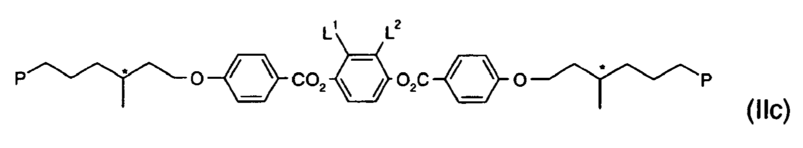

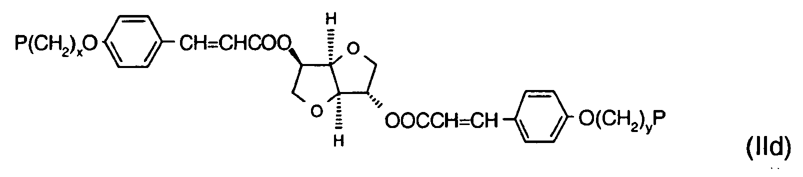

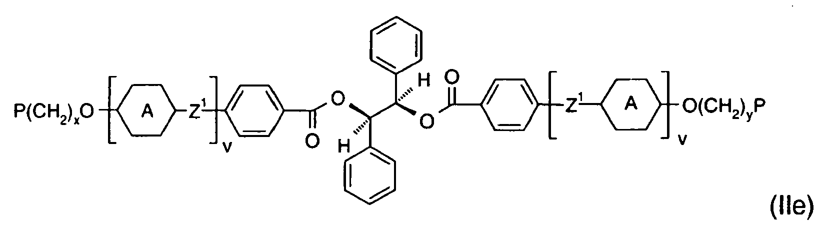

In the above formulae, P is a polymerizable group, preferably an acryl, methacryl, vinyl, vinyloxy, propenyl ether, epoxy or styryl group, x and y are each independently 1 to 12 , A is 1,4-phenylene that is optionally mono- di or trisubstituted by L¹ or 1,4-cyclohexylene, v is 0 or 1, Z⁰ is -COO-, -OCO-, -CH₂CH₂- or a single bond, Y is a polar group, Ter is a terpenoid radical like e.g. menthyl, Chol is a cholesteryl group, R⁰ is an unpolar alkyl or alkoxy group, and L¹ and L² are each independently H, F, Cl, CN or an optionally halogenated alkyl, alkoxy, alkylcarbonyl, alkoxycarbonyl or alkoxycarbonyloxy group with 1 to 7 C atoms.

The term 'polar group' in this connection means a group selected from F, Cl, CN, NO₂, OH, OCH₃, OCN, SCN, an optionally fluorinated carbonyl or carboxyl group with up to 4 C atoms or a mono- oligo- or polyfluorinated alkyl or alkoxy group with 1 to 4 C atoms. The term 'unpolar group' means an alkyl group with 1 or more, preferably 1 to 12 C atoms or an alkoxy group with 2 or more, preferably 2 to 12 C atoms.

In case chiral smectic or cholesteric LC materials are used, these preferably comprise a nematic or smectic host material and one or more chiral dopants that induce a helical twist in the host material. The chiral dopants can be polymerizable or not. They can be mesogenic or liquid crystal compounds, but do not necessarily have to be liquid crystalline.

Suitable polymerizable chiral compounds can be selected for example from the above formulae. Suitable non polymerizable chiral compounds can be selected e.g. from the commercially available chiral dopants R/S 811, R/S 1011, R/S 2011 or CB 15 (from Merck KGaA, Darmstadt, Germany). Very preferred are chiral dopants with a high helical twisting power (HTP), in particular those comprising a sorbitol group as described in WO 98/00428, those comprising a hydrobenzoin group as described in GB 2,328,207, chiral binaphthyl derivatives as described in EP 01111954.2, chiral binaphthol acetal derivatives as decribed in EP 01104842.8, chiral TADDOL derivatives as described in WO 02/06265, and chiral dopants with at least one fluorinated linkage group and a terminal or central chiral group as described in WO 02/06196 and WO 02/06195. The amount of chiral dopants in the liquid crystal material is preferably less than 15 %, in particular from 0.01 to 10 %, very preferably from 0.1 to 5 % by weight of the total liquid crystal material (without the solvent).

To prepare anisotropic polymer films, the polymerizable LC material is preferably coated onto a substrate, aligned and polymerized in situ, for example by exposure to heat or actinic radiation, to fix the orientation of the LC molecules. Alignment and curing are carried out in the LC phase of the material.

The polymerizable LC material can be applied onto the substate by conventional coating techniques like spin-coating or blade coating. It can also be applied to the substrate by conventional printing techniques which are known to the expert, like for example screen printing, offset printing, gravure printing or flexographic printing.

Suitable substrates include films, paper, board, leather, cellulose sheeting, textiles, plastics, glass, ceramics and metals. Suitable plastic substrates are for example films of polyester such as polyethyleneterephthalate (PET), polyvinylalcohol (PVA), polycarbonate (PC) or triacetylcellulose (TAC), especially preferably PET or TAC films. The substrate can be removed after polymerization or not.

The polymerizable chiral LC material may also be dissolved or dispersed in an organic solvent that is evaporated before or during polymerization. Preferably a solution is used. The solvent may evaporate with or without the use of external forces such as heat or reduced pressure.

Alignment of the LC material can be achieved for example by treatment of the substrate onto which the material is coated, by shearing the material during or after coating, by application of a magnetic or electric field to the coated material, or by the addition of surface-active compounds to the LC material. Reviews of alignment techniques are given for example by I. Sage in "Thermotropic Liquid Crystals", edited by G. W. Gray, John Wiley & Sons, 1987, pages 75-77, and by T. Uchida and H. Seki in "Liquid Crystals - Applications and Uses Vol. 3", edited by B. Bahadur, World Scientific Publishing, Singapore 1992, pages 1-63. A review of alignment materials and techniques is given by J. Cognard, Mol. Cryst. Liq. Cryst. 78, Supplement 1 (1981), pages 1-77.

Polymerization of the material takes place by exposing it to heat or actinic radiation. Actinic radiation means irradiation with light, like UV light, IR light or visible light, irradiation with X-rays or gamma rays or irradiation with high energy particles, such as ions or electrons. Preferably polymerization is carried out by UV irradiation. As a source for actinic radiation for example a single UV lamp or a set of UV lamps can be used. When using a high lamp power the curing time can be reduced. Another possible source for actinic radiation is a laser, like e.g. a UV laser, an IR laser or a visible laser.

In addition to the initiator of formula 14 the polymerizable material may comprise further initiators.

The polymerizable LC material can additionally comprise one or more other suitable components or additives such as, for example, catalysts, sensitizers, stabilizers, inhibitors, co-reacting monomers, surface-active compounds, lubricating agents, wetting agents, dispersing agents, hydrophobing agents, adhesive agents, flow improvers, defoaming agents, deaerators, diluents, reactive diluents, auxiliaries, colourants, dyes or pigments.

It is also possible, in order to increase crosslinking of the polymers, to add up to 20% of a non mesogenic compound with two or more polymerizable groups to the polymerizable LC material alternatively or in addition to the di- or multireactive polymerizable mesogenic compounds to increase crosslinking of the polymer. Typical examples for direactive non mesogenic monomers are alkyl diacrylates or alkyl dimethacrylates with alkyl groups of 1 to 20 C atoms. Typical examples for non mesogenic monomers with more than two polymerizable groups are trimethylpropane trimethacrylate or pentaerythritol tetraacrylate.

In another preferred embodiment the polymerizable LC material comprises up to 70%, preferably 3 to 50 % of a non mesogenic compound with one polymerizable group. Typical examples for monoreactive non mesogenic monomers are alkyl acrylates or alkyl methacrylates with alkyl groups of 1 to 20 C atoms.

It is also possible to add, for example, a quantity of up to 20% by weight of a non polymerizable LC compound to adapt the optical properties of the resulting polymer film.

The liquid crystal polymer film obtained by the inventive process can be used in optical elements, like reflective polarizers, retardation films, compensators, colour filters or holographic elements, especially in films with patterned optical properties, for the preparation of liquid crystal pigments, in decorative and security applications, like security markings that are applied to items or documents of value for easy identification or prevention of falsification, in nonlinear optics, optical recording or information storage.

The following examples are intended to illustrate the present invention. They do, however, not limit it in any way.

### Example 1

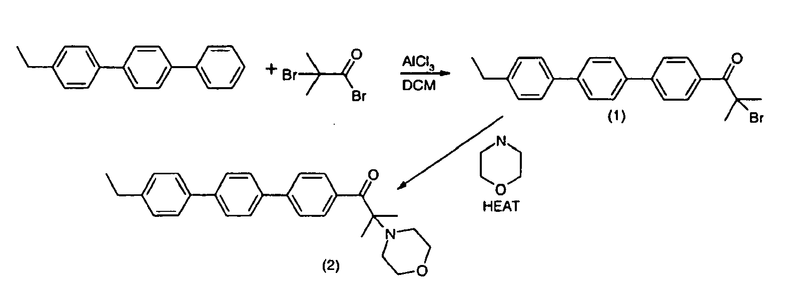

### 1.1 Synthesis of 2-Bromo-1-(4-ethyl-[1,1';4l,1"]terphenyl-4"-yl)-2-methyl-propan-1-one (1)

A suspension of AlCl₃ (16.001 g, 0.120 mol) in DCM (50 ml) was stirred at 0°C under nitrogen. 4-Ethyl-[1,1';4',1"]terphenyl (30.000 g, 0.116 mol) in DCM was added dropwise and stirred at 0°C for 20 min Bromoisobutyryl bromide (14.8 ml, 0.120 mol) was added dropwise, and the reaction left overnight at room temperature. The reaction mixture was then added in portions to a mixture of HCI and ice with stirring. The product was extracted into DCM (3 × 100 ml) and the combined organic layers were washed with water, brine, dried (Na₂SO₄) and the solvent removed under reduced pressure. The crude product was recrystallised from ethanol and used as is. The yield was 80% (37.8 g).

### 1.2 Synthesis of 1-(4-Ethyl-[1,1';4',1"]terphenyl-4"-yl)-2-methyl-2-morpholin-4-yl-propan-1-one (2)

2-Bromo-1- (4-ethyl- [1,1'; 4',1"]terphenyl-4"-yl)-2-methyl-propan-1-one (8.3 g, 0.020 mol) was added to stirring morpholine (100 ml) under nitrogen, at room temperature. The mixture was heated to 125°C and left to reflux for approximately 48 h. The mixture was allowed to cool to room temperature and stirred for a further 24 h, then added to water portionwise. The organic layer was washed with water (3 × 20 ml) and brine (1 × 20 ml), then dried (Na₂SO₄) and the solvent removed under reduced pressure.

The crude product was purified by column chromatography using a 50/50 mix of DCM/Petrol 40:60 to yield pure product. Yield 51% (4.22 g). MPt 180°C, NMR and MS were performed to confirm the structure.

### Example 2- Comparative Example

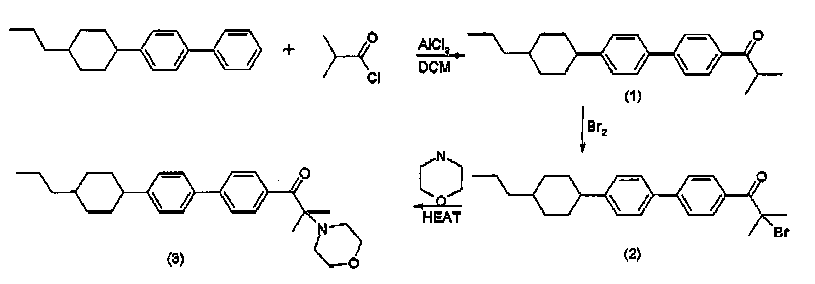

### 2.1 Synthesis of 2-Methyl-1-[4'-(4-propyl-cyclohexyl)-biphenyl-4-yl]-propan-1-one (1)

A suspension of AICl₃ (14.401 g, 0.108 mol) in DCM (50 ml) was stirred at 0°C under nitrogen 4-(4-Propyl-cyclohexyl)-biphenyl (30 g, 0.108 mol) in DCM was added dropwise and stirred at 0°C for 20 min. Isobutyryl chloride (~11.5 ml, ~0.108 mol) was added dropwise, and the reaction left overnight at room temperature. The reaction mixture was then added in portions to a mixture of HCI and ice with stirring. The product was extracted into DCM (3 × 100 ml) and the combined organic layers were washed with water, brine, dried (Na₂SO₄) and the solvent removed under reduced pressure. The crude product was recrystallised from ethanol and used as is.
Yield 86% (32.0 g)

### 2.2 Synthesis of 2-Bromo-2-methyl-1-[4'-(4-propyl-cyclohexyl)-biphenyl-4-yl]-propan-1-one (2)

Bromine (17.579 g, 0.110 mol) was added dropwise to a stirred mixture of 2-Methyl-1-[4'-(4-propyl-cyclohexyl)-biphenyl-4-yl]-propan-1-one (32.0 g, 0.093 mol) in DCM at 0°C under nitrogen. The reaction mixture was allowed to warm to room temperature and left stirring overnight. The mixture was then diluted with DCM, washed with sodium metabisulphite solution (50 ml × 2), water, dried (Na₂SO₄) and the solvent removed under reduced pressure. The crude product was purified by recrystallisation from ethanol.
Yield 83% (32.9 g)

### 2.3. Synthesis of 2-Methyl-2-morpholin-4-yl-1-[4'-(4-propylcyclohexyl)-biphenyl-4-yl]-propan-1-one (3)

2-Bromo-2-methyl-1-[4'-(4-propyl-cyclohexyl)-biphenyl-4-yl]-propan-1-one (32.9 g, 0.077 mol)was added to stirring morpholine under nitrogen, at room temperature. The mixture was heated to 125°C and left to reflux for approximately 48 h. The mixture was allowed to cool to room temperature and stirred for a further 24 h, then added to water portionwise. The organic layer was washed with water (3 × 20 ml) and brine (1 × 20 ml), then dried (Na₂SO₄) and the solvent removed under reduced pressure. The crude product was purified by column chromatography using a 50/50 mix of DCM/Petrol 40:60 to yield pure product.
Yield 46% (15.4 g). MPt 145°C. NMR and MS were performed to confirm the structure.

### Example 3

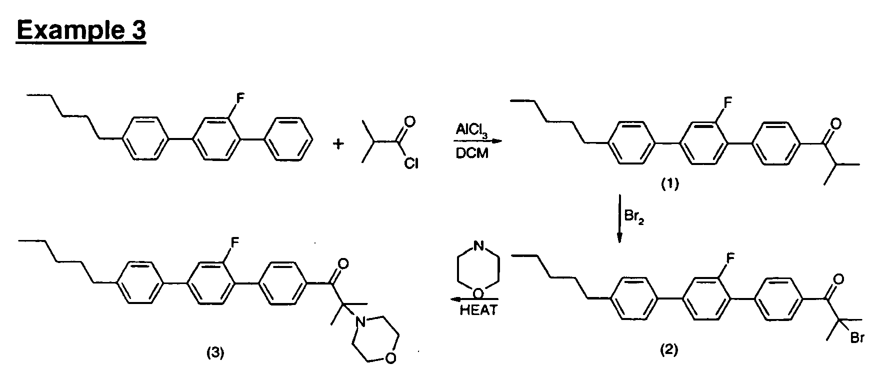

### 3.1 Synthesis of 1-(2'-Fluoro-4"-pentyl-[1,1';4',1"]terphenyl-4-yl)-2-methyl-propan-1-one (1)

A suspension of AlCl₃ (14.401 g, 0.108 mol) in DCM (50 ml) was stirred at 0°C under nitrogen. 2'-Fluoro-4"-pentyl-[1,1';4',1"]terphenyl (34 g, 0.108 mol) in DCM was added dropwise and stirred at 0°C for 20 min. Isobutyryl chloride (~11.5 ml, ~0.108 mol) was added dropwise, and the reaction left overnight at room temperature. The reaction mixture was then added in portions to a mixture of HCI and ice with stirring. The product was extracted into DCM (3 × 100 ml) and the combined organic layers were washed with water, brine, dried (Na₂SO₄) and the solvent removed under reduced pressure. The crude product was recrystallised from ethanol and used as is.
Yield 90% (45.4 g)

### 3.2 Synthesis of 2-Bromo-1-(2'-fluoro-4"-pentyl-[1,1';4',1"]terphenyl-4-yl)-2-methyl-propan-1-one (2)

Bromine (17.579 g, 0.110 mol) was added dropwise to a stirred mixture of 1-(2'-Fluoro-4"-pentyl-[1,1';4',1"]terphenyl-4-yl)-2-methyl-propan-1-one (42.680 g, 0.110 mol) in DCM at 0°C under nitrogen. The reaction mixture was allowed to warm to room temperature and left stirring overnight. The mixture was then diluted with DCM, washed with sodium metabisulphite solution (50 ml × 2), water, dried (Na₂SO₄) and the solvent removed under reduced pressure. The crude product was purified by recrystallisation from ethanol.
Yield 74% (37.8 g)

### 3.3 Synthesis of 1-(2'-Fluoro-4"-pentyl-[1,1';4',1"]terphenyl-4-yl)-2-methyl-2-morpholin-4-yl-propan-1-one (3)

2-Bromo-1-(2'-fluoro-4"-pentyl-[1,1';4',1"]terphenyl-4-yl)-2-methylpropan-1-one (37.8 g, 0.097 mol) was added to stirring morpholine under nitrogen, at room temperature. The mixture was heated to 125°C and left to reflux for approximately 48 h. The mixture was allowed to cool to room temperature and stirred for a further 24 h, then added to water portionwise. The organic layer was washed with water (3 × 20 ml) and brine (1 × 20 ml), then dried (Na₂SO₄) and the solvent removed under reduced pressure. The crude product was purified by column chromatography using a 50/50 mix of DCM/Petrol 40:60 to yield pure product.
Yield 55% (25.2 g). MPt 148°C. NMR and MS were prerformed to confirm the structure.

### Example 4

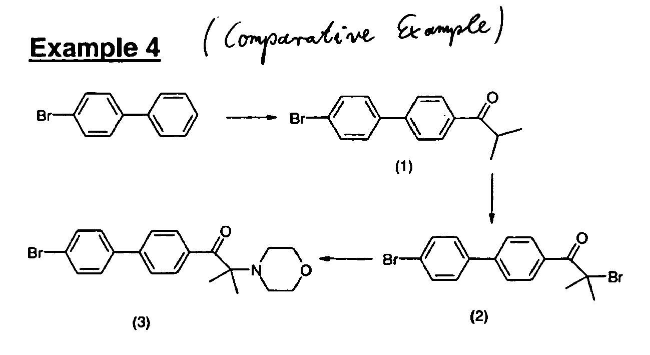

### 4.1 Synthesis of 1-(4'-Bromo-biphenyl-4-yl)-2-methyl-propan-1-one (1)

A suspension of AlCl₃ (5.491 g, 0.041 mol) in DCM (50 ml) was stirred at 0°C under nitrogen. 4-Bromo-biphenyl (8 g, 0.034 mol) in DCM (20 ml) was added dropwise and stirred at 0°C for 20 min. Isobutyryl chloride (4.315 ml, 0.041 mol) was added dropwise, and the reaction left overnight at room temperature. The reaction mixture was then added in portions to a mixture of HCI and ice with stirring. The product was extracted into DCM (3 × 100 ml) and the combined organic layers were washed with water, brine, dried (Na₂SO₄) and the solvent removed under reduced pressure. The crude product was recrystallised from ethanol and used as is.
Yield 90% (9.42 g)

### 4.2 Synthesis of 2-Bromo-1-(4'-bromo-biphenyl-4-yl)-2-methylpropan-1-one (2)

Bromine (1.00 ml, 0.019 mol) was added dropwise to a stirred mixture of 1-(4'-Bromo-biphenyl-4-yl)-2-methyl-propan-1-one (5.40 g, 0.017 mol) in DCM at 0°C under nitrogen. The reaction mixture was allowed to warm to room temperature and left stirring overnight. The mixture was then diluted with DCM, washed with sodium metabisulphite solution (50 ml × 2, or until neutral), water, brine, then dried (Na₂SO₄) and the solvent removed under reduced pressure. The crude product was purified by recrystallisation from ethanol to give an off-white solid.
Yield 74% (5.00 g)

### 4.3 Synthesis of 1-(4'-Bromo-biphenyl-4-yl)-2-methyl-2-morpholin-4-yl-propan-1-one (3)

2-Bromo-1-(4'-bromo-biphenyl-4-yl)-2-methyl-propan-1-one (5.00 g, 0.013 mol) was added to stirring morpholine (75 ml) under nitrogen, at room temperature. The mixture was heated to 145°C and left to reflux for approximately 19 h. The mixture was allowed to cool to room temperature and stirred for a further 24 h, then added to water portionwise. The organic layer was washed with water (3 × 20 ml) and brine (1 × 20 ml), then dried (Na₂SO₄) and the solvent removed under reduced pressure. The crude product was purified by column chromatography using a 50/50 mix of DCM/Petrol 40:60 to yield pure product. Yield 40% (2.05 g). Mpt 136.5°C. NMR and MS were perfomed to confirm the structure.

### Example 5

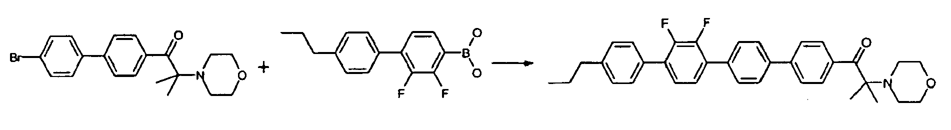

### Synthesis of 1-(2",3"-Difluoro-4"'-propyl-[1,4';1',1";4",1"']quaterphenyl-4-yl)-2-methyl-2-morpholin-4-yl-propan-1-one

A solution of sodium carbonate (0.082 g, 0.007 mol) in water (5 ml) was added to a solution of 2,3-Difluoro-4'-propyl-biphenylboronic acid (0.213 g, 0.007 mol) in THF (5 ml), followed by addition of 1-(4'-Bromo-biphenyl-4-yl)-2-methyl-2-morpholin-4-yl-propan-1-one (0.300 g, 0.007 mol) and Tetrakistriphenyl phosphine palladium (0) catalyst (0.010 g). The mixture was heated to 80°C and left to reflux under nitrogen for 18 h, then allowed to cool to room temperature. The product was extracted into DCM (3 × 100 ml) and the combined organic layers were washed with water, brine, dried (Na₂SO₄) and the solvent removed under reduced pressure. The resultant light yellow solid was recrystallised from ethanol to yield the product as a white solid.
Yield 41% (1.71 g). MPt 196°C. NMR and MS was perfomed to confirm the structure.

### Example 6

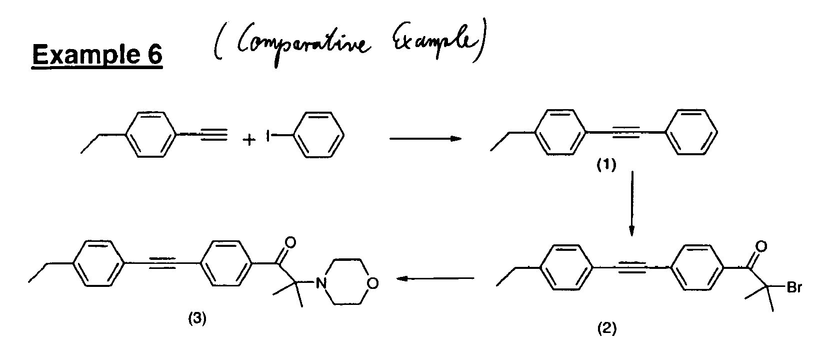

### 6. 1 Synthesis of 1 -Ethyl-4-phenylethynyl-benzene (1)

A solution of 1-Ethyl-4-ethynyl-benzene (0.702 g, 0.054 mol) in THF (40 ml) was added to a stirred mixture of iodobenzene (1.000 g, 0.049 mol), dichlorobis(triphenylphosphine)-palladium(II) (0.344 g, 0.00490 mol), and copper iodide (0.020 g) with triethylamine (20 ml) under nitrogen. The mixture was heated to 85°C and left to stir for 19 h, then cooled to room temperature. The mixture was added to water and extracted using DCM, the organics were washed with water (2 × 50 ml), brine (1 × 50 ml), dried over sodium sulphate, filtered and the excess solvent evaporated under reduced pressure. The resultant solid was purified by column chromatography using a 50/50 mix of DCM/Petrol 40:60, and recrystallised from ethanol to give pure product.
Yield 90% (0.91 g)

### 6.2 Synthesis of 2-Bromo-1-[4-(4-ethyl-phenylethynyl)-phenyl]-2-methyl-propan-1-one (2)

A solution of 1-Ethyl-4-phenylethynyl-benzene (400 mg, 1.939 mmol) in anhydrous DCM (10 ml) was added dropwise to a suspension of aluminium chloride (310 mg, 2.327 mmol) in anhydrous DCM (10 ml) at 0°C under nitrogen. The bromoisobutyryl bromide (0.288 ml, 2.327 mmol) was added dropwise, the mixture allowed to warm to room temperature and left to stir for 18 h. The reaction mixture was then added in portions to a mixture of HCI and ice with stirring. The product was extracted into DCM (3 × 100 ml) and the combined organic layers were washed with water, brine, dried (Na₂SO₄) and the solvent removed under reduced pressure. The crude product was recrystallised from ethanol and used as is.
Yield 84% (0.58 g)

### 6.3 Synthesis of 1-[4-(4-Ethyl-phenylethynyl)-phenyl]-2-methyl-2-morpholin-4-yl-propan-1-one (3)

2-Bromo-1-[4-(4-ethyl-phenylethynyl)-phenyl]-2-methyl-propan-1-one (0.58 g, 0.0016 mol) was added to stirring morpholine (75 ml) under nitrogen, at room temperature. The mixture was heated to 145°C and left to reflux for approximately 19 h. The mixture was allowed to cool to room temperature and stirred for a further 24 h, then added to water portionwise. The organic layer was washed with water (3 × 20 ml) and brine (1 × 20 ml), then dried (Na₂SO₄) and the solvent removed under reduced pressure. The crude product was purified by column chromatography using a 50/50 mix of DCM/Petrol 40:60 to yield pure product. Yield 48% (0.28 g). NMR and MS confirmed structure.

### Example 7

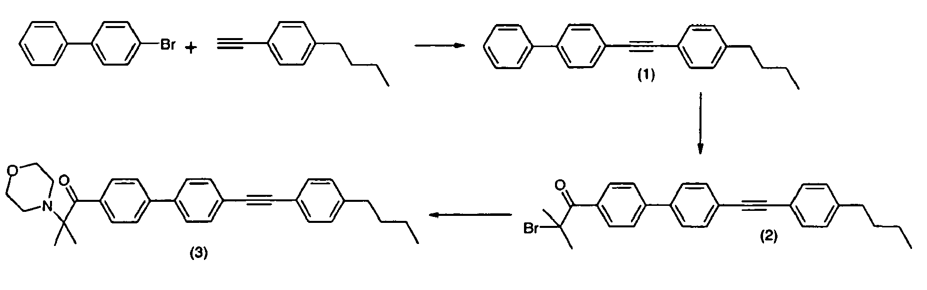

### 7.1 Synthesis of 4-(4-Butyl-phenylethynyl)-biphenyl (1)

A solution of 1-butyl-4-ethynyl-benzene (0.747 g, 4.719 mmol) in THF (20 ml) was added to a stirred mixture of 4-Bromo-biphenyl (1.000 g, 4.290 mmol), dichlorobis(triphenylphosphine)-palladium(ll) (0.301 g, 0.429 mmol), and copper iodide (0.010 g) with triethylamine (10 ml) under nitrogen. The mixture was heated to 85°C and left to stir for 19 h in reduced light, then cooled to room temperature. The mixture was added to water and extracted using DCM, the organics were washed with water (2 × 50 ml), brine (1 × 50 ml), dried over sodium sulphate, filtered and the excess solvent evaporated under reduced pressure. The resultant solid was purified by column chromatography using a 50/50 mix of DCM/Petrol 40:60, and recrystallised from ethanol to give pure product.
Yield 88% (1.2 g)

### 7.2.Synthesis of 2-Bromo-1-[4'-(4-butyl-phenylethynyl)-biphenyl-4-yl]-2-methyl-propan-1-one (2)

A solution of 4-(4-Butyl-phenylethynyl)-biphenyl (1.2 g, 3.865 mmol) in anhydrous DCM (10 ml) was added dropwise to a suspension of aluminium chloride (0.53 g, 4.0 mmol) in anhydrous DCM (10 ml) at 0°C under nitrogen. The bromoisobutyryl bromide (0.5 ml, 4.0 mmol) was added dropwise, the mixture allowed to warm to room temperature and left to stir for 18 h. The reaction mixture was then added in portions to a mixture of HCI and ice with stirring. The product was extracted into DCM (3 × 100 ml) and the combined organic layers were washed with water, brine, dried (Na₂SO₄) and the solvent removed under reduced pressure. The crude product was recrystallised from ethanol and used as is.
Yield 89% (1.58 g)

### 7.3 Synthesis of 1-[4'-(4-Butyl-phenylethynyl)-biphenyl-4-yl]-2-methyl-2-morpholin-4-yl-propan-1-one (3)

2-Bromo-1-[4'-(4-butyl-phenylethynyl)-biphenyl-4-yl]-2-methylpropan-1-one (1.58 g, 3.45mmol) was added to stirring morpholine (75 ml) under nitrogen, at room temperature. The mixture was heated to 145°C and left to reflux for approximately 19 h. The mixture was allowed to cool to room temperature and stirred for a further 24 h, then added to water portionwise. The organic layer was washed with water (3 × 20 ml) and brine (1 × 20 ml), then dried (Na₂SO₄) and the solvent removed under reduced pressure. The crude product was purified by column chromatography using a 50/50 mix of DCM/Petrol 40:60 to yield pure product.
Yield 51% (0.8 g). NMR and MS was performed to confirm the structure.

## Claims

1. A compound of formula I4 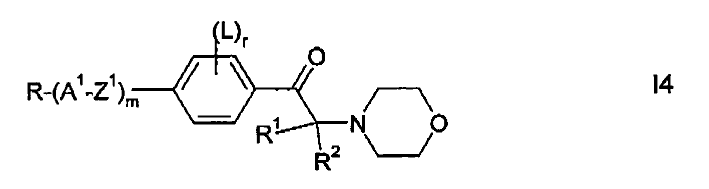 wherein
R is straight chain or branched alkyl with 1 to 20 C- atoms, which may be unsubstituted, mono- or poly- substituted by F, Cl, Br, I or CN, it being also possible for one or more non-adjacent CH₂ groups to be replaced, in each case independently from one another, by -O-, -S-, -NH-, -NR⁰-, -SiR⁰R⁰⁰-, - CO-, -COO-, -OCO-, -OCO-O-, -S-CO-, -CO-S-, - CH=CH- or -C≡C- in such a manner that O and/or S atoms are not linked directly to one another,
R⁰ and R⁰⁰ are independently of each other H or alkyl with 1 to 4 C atoms,
R¹ and R² are methyl or ethyl, wherein -CR¹R²- is different from -C(CH₃)(C₂H₅)-,
A¹ is 1,4-phenylene, which is unsubstituted, or mono- or polysubstituted by L,
L is F,
r is 0,1, 2, 3 or 4,
Z¹ is -C≡C- or a single bond, and
m is 2, 3 or 4.

2. A compound according to claim 1, wherein the group -(A¹-Z¹)ₘ- is selected from the following list of preferred groups, which is also comprising their mirror images, wherein Phe in these groups is 1,4-phenylene, Phe L is a 1,4-phenylene group which is substituted by 1 to 4 groups L as defined in claim 1,
- Phe-Z-Phe-Z- II-3
- PheL-Z-Phe-Z- II-6
- PheL-Z-PheL-Z- II-8
- Phe-Z-Phe-Z-Phe-Z- II1-9
- Phe-Z-Phe-Z-PheL-Z- II-15
- Phe-Z-PheL-Z-Phe-Z- II-16
- PheL-Z-Phe-Z-Phe-Z- II-17
- PheL-Z-Phe-Z-PheL-Z- II-18
- PheL-Z-PheL-Z-Phe-Z- II-19
- PheL-Z-PheL-Z-PheL-Z-II-20
and wherein Z in each case independently has one of the meanings of Z¹ as given in claim 1.

3. A compound according to claim 1 or 2, wherein R is an alkyl or alkoxy radical that is straight-chain and is ethyl, propyl, butyl, pentyl, hexyl, heptyl, octyl, ethoxy, propoxy, butoxy, pentoxy, hexoxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy.

4. A compound according to at least one of claims 1 to 3, wherein R is an alkyl or alkenyl group that is at least monosubstituted by halogen, is straight-chain, and halogen is F or Cl.

5. A compound according to claim 4, wherein R is multiple substituted by F.

6. A compound according to at least one of claims 1 to 5, selected of the following formulae 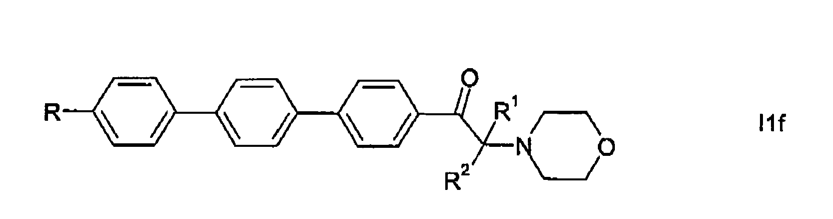 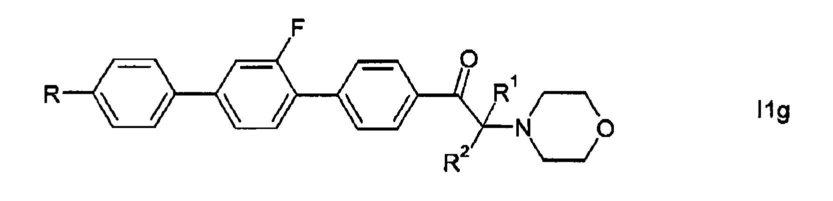 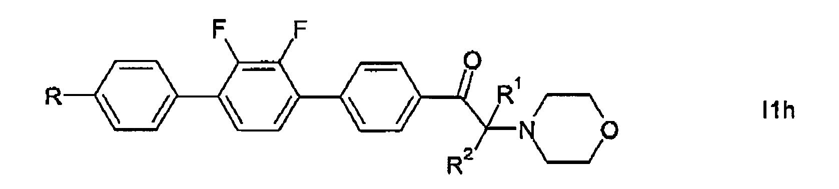 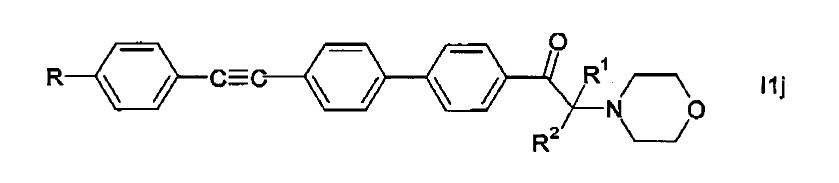 wherein R and R¹⁻² have the meanings given in claims 1 to 6.

7. A compound according to claim 6, wherein R is alkyl or alkoxy with 1 to 8 C-atoms.

8. Use of a compound according to at least one of claims 1 to 7 as photoinitiator.

9. Use according to claim 8 for the preparation of polymers or polymer films by polymerizing or copolymerizing a polymerizable liquid crystal composition comprising at least one compound of formula 14 according to at least one of claims 1 to 7, or by polymerizing one or more polymerizable compounds in the presence of at least one initiator of formula 14 according to at least one of claims 1 to 7.

10. Polymerizable composition comprising at least one compound according to at least one of claims 1 to 7 and at least one polymerizable compound.

11. Polymerizable composition according to claim 10, **characterized in that** it is a liquid crystal composition.

12. Polymerizable composition according to claim 10 or 11, **characterized in that** it comprises 0.01 to 10% of an initiator of formula 14 according to one or more of claims 1 to 7.

13. Polymerizable composition according to at least one of claims 10 to 12, **characterized in that**, in addition to the initiator of formula 14, it comprises further initiators.

14. Polymerizable composition according to at least one of claims 10 to 13, **characterized in that** it comprises at least one polymerizable mesogenic compound having one polymerizable functional group and at least one polymerizable mesogenic compound having two or more polymerizable functional groups.

15. Polymerizable composition according to at least one of claims 10 to 14, **characterized in that** the polymerizable compounds are selected from the following formulae 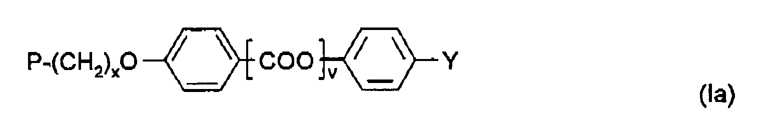 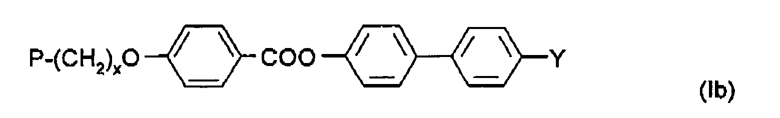 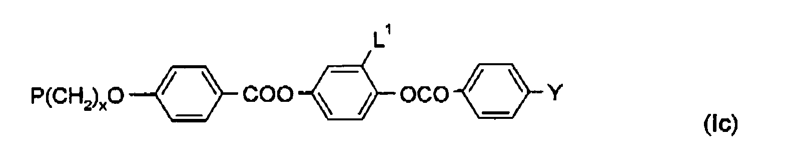 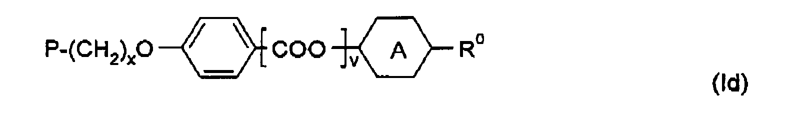 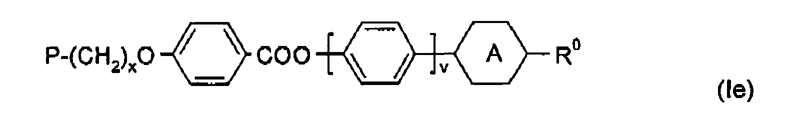 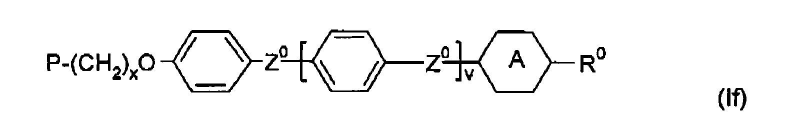 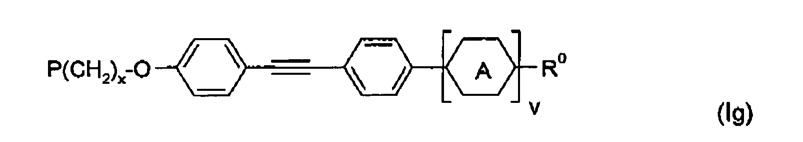 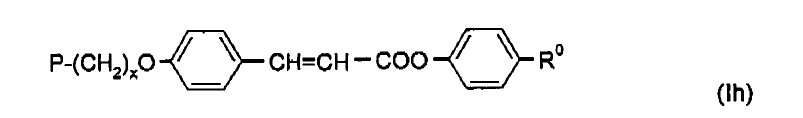 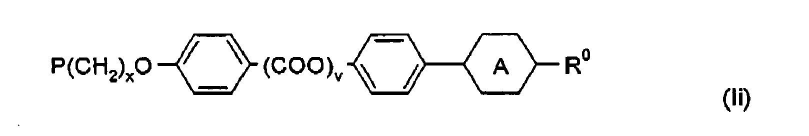 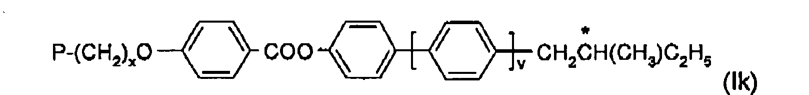 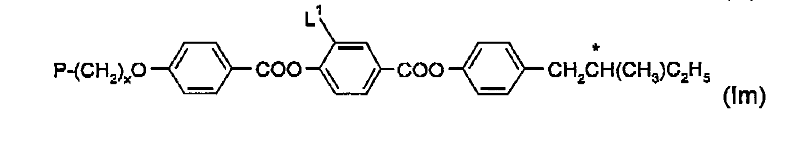 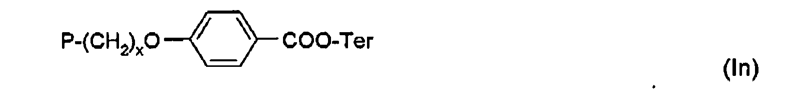 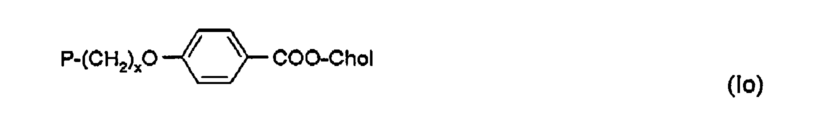 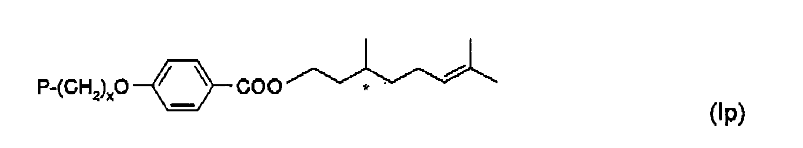 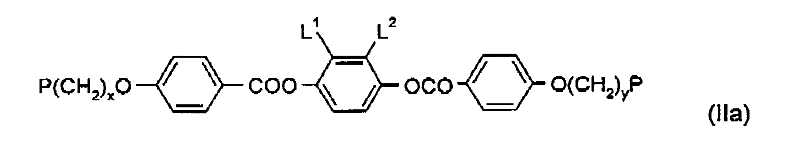 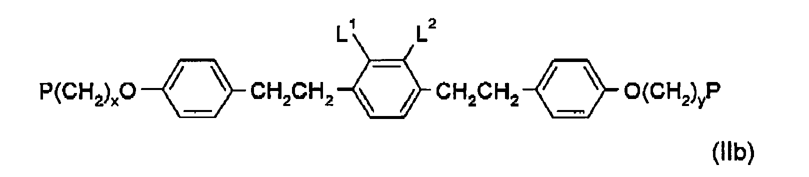 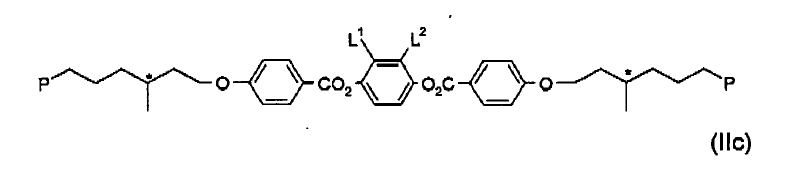 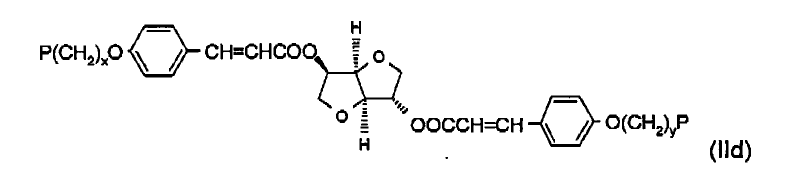 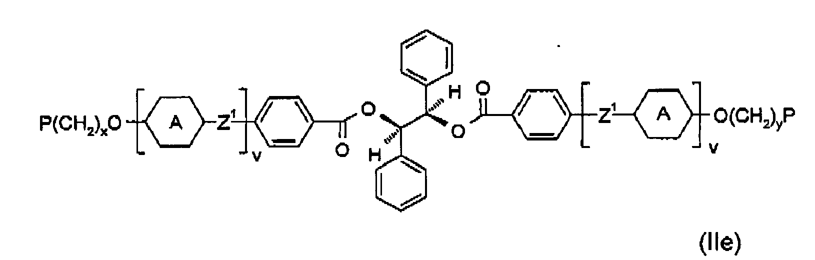 wherein
P is an acryl, methacryl, vinyl, vinyloxy, propenyl ether, epoxy or styryl group,
x and y are each independently 1 to 12,
A is 1,4-phenylene that is optionally mono- di or trisubstituted by L¹ or 1,4-cyclohexylene,
v is 0 or 1,
Z⁰ is -COO-, -OCO-, -CH₂CH₂- or a single bond,
Y is a group selected from F, Cl, CN, NO₂, OH, OCH₃, OCN, SCN, an optionally fluorinated carbonyl or carboxyl group with up to 4 C atoms or a mono- oligo- or polyfluorinated alkyl or alkoxy group with 1 to 4 C atoms,
Ter is a terpenoid radical,
Chol is a cholesteryl group,
R⁰ is an alkyl group with 1 to 12 C atoms or an alkoxy group with 2 to 12 C atoms, and
L¹ and L² are each independently H, F, Cl, CN or an optionally halogenated alkyl, alkoxy, alkylcarbonyl, alkoxycarbonyl or alkoxycarbonyloxy group with 1 to 7 C atoms.

16. Polymer obtained by polymerizing one or more polymerizable compounds in the presence of at least one compound according to at least one of claims 1 to 7 or by polymerizing a composition according to at least one of claims 10 to 15.

17. Anisotropic polymer film obtained by polymerizing a polymerizable liquid crystal composition according to at least one of claims 10 to 15 that is oriented in its liquid crystal phase.

18. Use of a compound, composition, polymer or polymer film according to at least one of claims 1 to 7 and 10 to 17 in liquid crystal displays, optical films, polarisers, compensators, beam splitters, reflective films, alignment layers, colour filters, holographic elements, hot stamping foils, coloured images, decorative or security markings, liquid crystal pigments, adhesives, synthetic resins with anisotropic mechanical properties, cosmetics, diagnostics, nonlinear optics, optical information storage, as chiral dopants, in electronic devices like field effect transistors (FET) as components of integrated circuitry, as thin film transistors in flat panel display applications or for Radio Frequency Identification (RFID) tags, in semiconducting components for organic light emitting diode (OLED) applications, electroluminescent displays or backlights of liquid crystal displays, for photovoltaic or sensor devices, as electrode materials in batteries, as photoconductors, for electrophotographic applications or electrophotographic recording.

## Patentansprüche

1. Verbindung der Formel I4 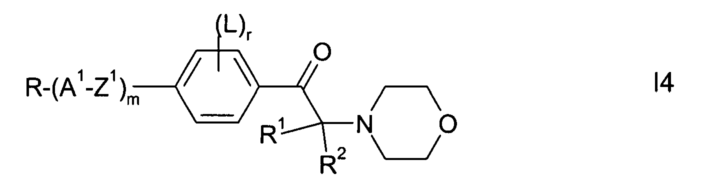 worin
R geradkettiges oder verzweigtes Alkyl mit 1 bis 20 C- Atomen bedeutet, das unsubstituiert oder ein- oder mehrfach durch F, Cl, Br, I oder CN substituiert sein kann, wobei auch eine oder mehrere nicht benach- barte CH₂-Gruppen jeweils unabhängig vonein- ander so durch -O-, -S-, -NH-, -NR⁰-, -SiR⁰R⁰⁰-, -CO-, -COO-, -OCO-, -OCO-O-, -S-CO-, -CO-S-, -CH=CH- oder -C=C- ersetzt sein können, dass O- und/oder S-Atome nicht direkt miteinander ver- knüpft sind,
R⁰ und R⁰⁰ unabhängig voneinander H oder Alkyl mit 1 bis 4 C- Atomen bedeuten,
R¹ und R² Methyl oder Ethyl bedeuten, worin -CR¹R²- von -C(CH₃)(C₂H₅)- verschieden ist,
A¹ 1,4-Phenylen bedeutet, das unsubstituiert oder ein- oder mehrfach durch L substituiert ist,
L F bedeutet,
r für 0,1, 2, 3 oder 4 steht,
Z¹ -C≡C- oder eine Einfachbindung bedeutet und
m für 2, 3 oder 4 steht.

2. Verbindung nach Anspruch 1, worin die Gruppe -(A¹-Z¹)ₘ- ausgewählt ist aus der folgenden Liste bevorzugter Gruppen, die auch deren Spiegelbilder umfasst, worin Phe in diesen Gruppen 1,4-Phenylen bedeutet, Phe L eine 1,4-Phenylengruppe bedeutet, die durch 1 bis 4 Gruppen L wie in Anspruch 1 definiert substituiert ist,
- Phe-Z-Phe-Z- II-3
- PheL-Z-Phe-Z- II-6
- PheL-Z-PheL-Z- II-8
- Phe-Z-Phe-Z-Phe-Z- II-9
- Phe-Z-Phe-Z-PheL-Z- II-15
- Phe-Z-PheL-Z-Phe-Z- II-16
- PheL-Z-Phe-Z-Phe-Z- II-17
- PheL-Z-Phe-Z-PheL-Z- II-18
- PheL-Z-PheL-Z-Phe-Z- II-19
- PheL-Z-PheL-Z-PheL-Z- II-20
und worin Z jeweils unabhängig eine der Bedeutungen von Z¹ wie in Anspruch 1 angegeben besitzt.

3. Verbindung nach Anspruch 1 oder 2, worin R einen Alkyl- oder Alkoxyrest bedeutet, der geradkettig ist und Ethyl, Propyl, Butyl, Pentyl, Hexyl, Heptyl, Octyl, Ethoxy, Propoxy, Butoxy, Pentoxy, Hexoxy, Heptoxy oder Octoxy, weiterhin Methyl, Nonyl, Decyl, Undecyl, Dodecyl, Tridecyl, Tetradecyl, Pentadecyl, Nonoxy, Decoxy, Undecoxy, Dodecoxy, Tridecoxy oder Tetradecoxy bedeutet.

4. Verbindung nach mindestens einem der Ansprüche 1 bis 3, worin R eine Alkyl- oder Alkenylgruppe bedeutet, die mindestens einfach durch Halogen substituiert und geradkettig ist, und Halogen F oder Cl bedeutet.

5. Verbindung nach Anspruch 4, worin R mehrfach durch F substituiert ist.

6. Verbindung nach mindestens einem der Ansprüche 1 bis 5, ausgewählt aus den folgenden Formeln 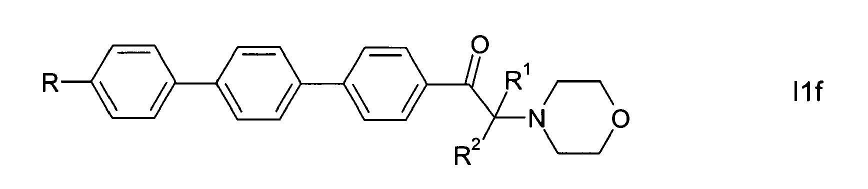 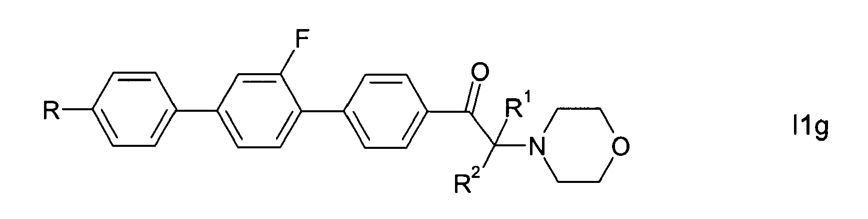 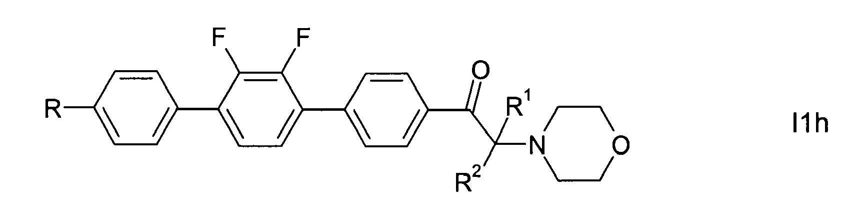 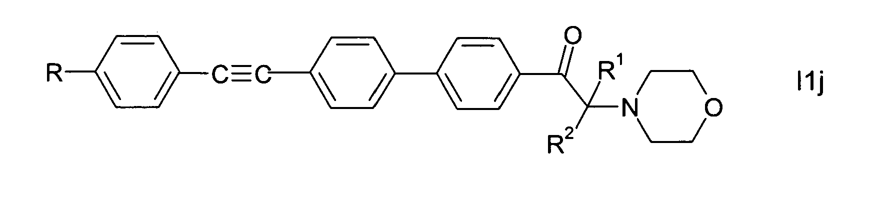 worin R und R¹⁻² die in den Ansprüchen 1 bis 5 angegebenen Bedeutungen besitzen.

7. Verbindung nach Anspruch 6, worin R Alkyl oder Alkoxy mit 1 bis 8 C-Atomen bedeutet.

8. Verwendung einer Verbindung nach mindestens einem der Ansprüche 1 bis 7 als Photoinitiator.

9. Verwendung nach Anspruch 8 zur Herstellung von Polymeren oder Polymerfolien durch Polymerisation oder Copolymerisation einer polymerisierbaren Flüssigkristallzusammensetzung enthaltend mindestens eine Verbindung der Formel I4 nach mindestens einem der Ansprüche 1 bis 7 oder durch Polymerisation einer oder mehrerer polymerisierbarer Verbindungen in Gegenwart mindestens eines Initiators der Formel I4 nach mindestens einem der Ansprüche 1 bis 7.

10. Polymerisierbare Zusammensetzung enthaltend mindestens eine Verbindung nach mindestens einem der Ansprüche 1 bis 7 und mindestens eine polymerisierbare Verbindung.

11. Polymerisierbare Zusammensetzung nach Anspruch 10,
**dadurch gekennzeichnet, dass** es sich um eine Flüssigkristallzusammensetzung handelt.

12. Polymerisierbare Zusammensetzung nach Anspruch 10 oder 11, **dadurch gekennzeichnet, dass** sie 0,01 bis 10% eines Initiators der Formel I4 nach einem oder mehreren der Ansprüche 1 bis 7 enthält.

13. Polymerisierbare Zusammensetzung nach mindestens einem der Ansprüche 10 bis 12, **dadurch gekennzeichnet, dass** sie zusätzlich zum Initiator der Formel I4 weitere Initiatoren enthält.

14. Polymerisierbare Zusammensetzung nach mindestens einem der Ansprüche 10 bis 13, **dadurch gekennzeichnet, dass** sie mindestens eine polymerisierbare mesogene Verbindung mit einer polymerisierbaren funktionellen Gruppe und mindestens eine polymerisierbare mesogene Verbindung mit zwei oder mehr polymerisierbaren funktionellen Gruppen enthält.

15. Polymerisierbare Zusammensetzung nach mindestens einem der Ansprüche 10 bis 14, **dadurch gekennzeichnet, dass** die polymerisierbaren Verbindungen ausgewählt sind aus den folgenden Formeln 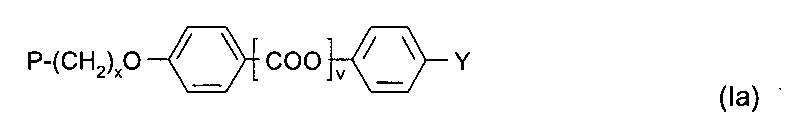 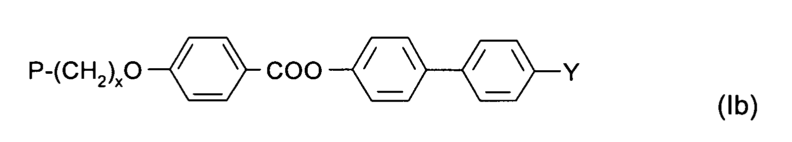 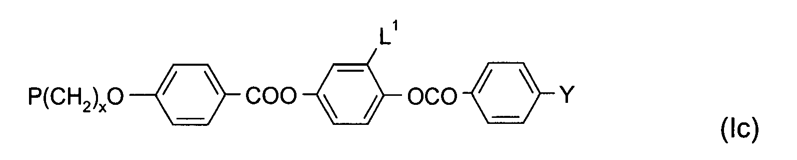 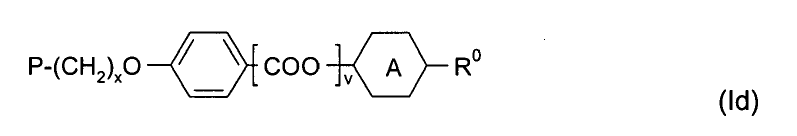 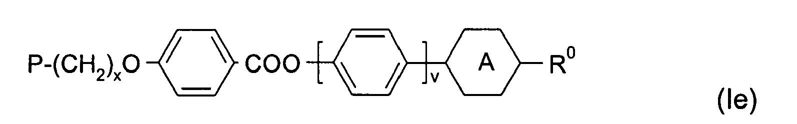 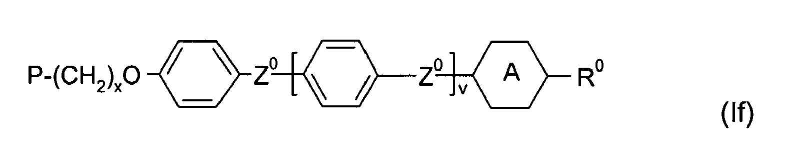 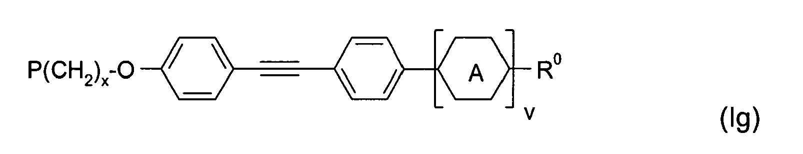 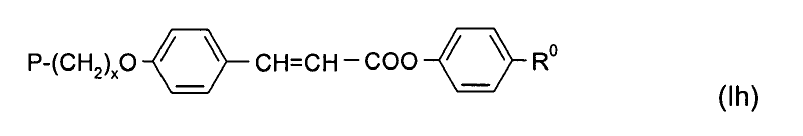 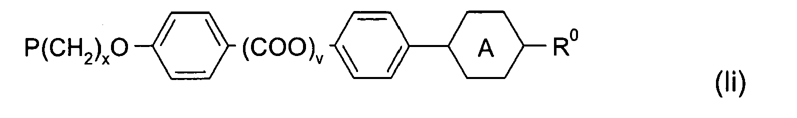 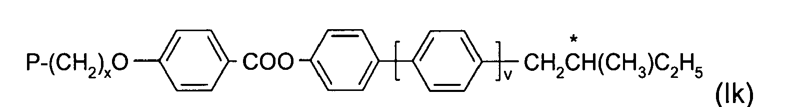 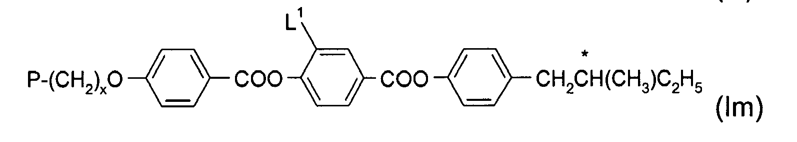 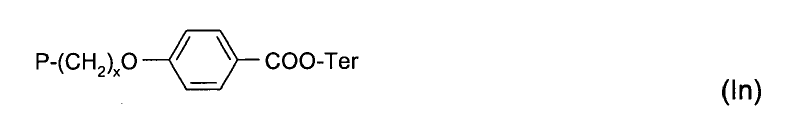 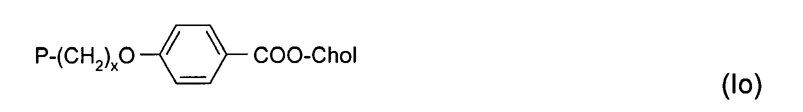 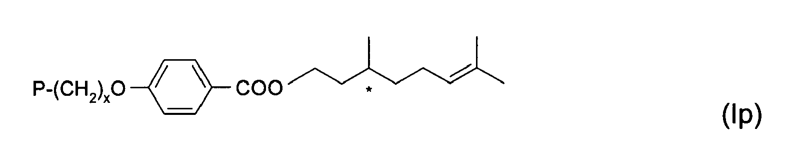 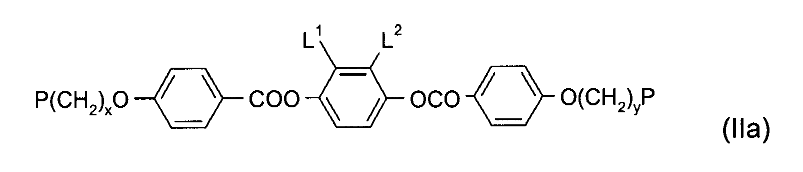 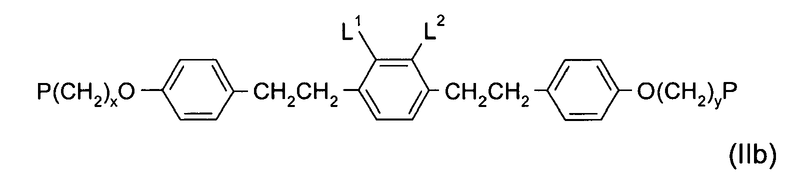 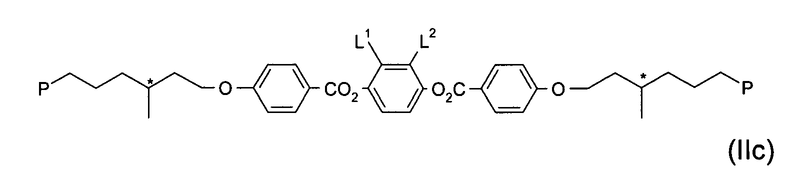 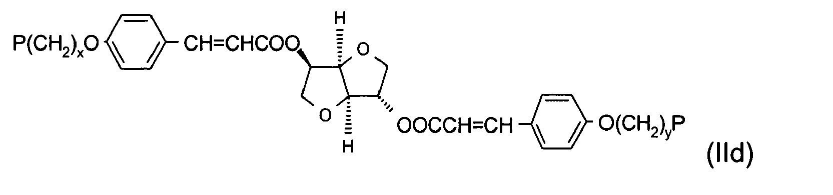 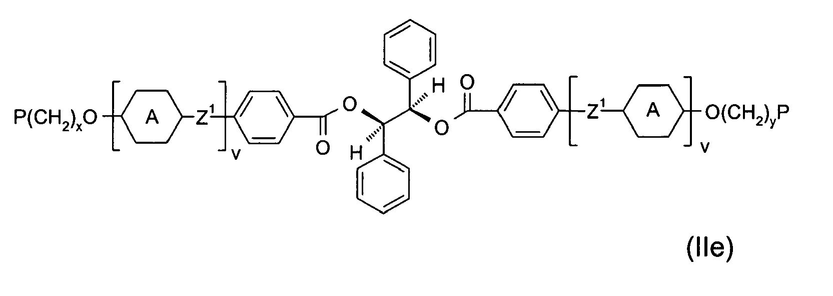 worin
P eine Acryl-, Methacryl-, Vinyl-, Vinyloxy-, Propenylether-, Epoxy- oder Styrylgruppe bedeutet,
x und y jeweils unabhängig für 1 bis 12 stehen,
A 1,4-Phenylen bedeutet, das gegebenenfalls ein-, zwei- oder dreifach durch L¹ oder 1,4-Cyclohexylen substituiert ist,
v für 0 oder 1 steht,
Z⁰ -COO-, -OCO-, -CH₂CH₂- oder eine Einfachbindung bedeutet,
Y eine Gruppe ausgewählt aus F, Cl, CN, NO₂, OH, OCH₃, OCN, SCN, eine gegebenenfalls fluorierte Carbonyl- oder Carboxylgruppe mit bis zu 4 C-Atomen oder eine mono-, oligo- oder polyfluorierte Alkyl- oder Alkoxygruppe mit 1 bis 4 C-Atomen bedeutet,
Ter einen Terpenoidrest bedeutet,
Choleine Cholesterylgruppe bedeutet,
R⁰ eine Alkylgruppe mit 1 bis 12 C-Atomen oder eine Alkoxy- gruppe mit 2 bis 12 C-Atomen bedeutet und
L¹ und L² jeweils unabhängig H, F, Cl, CN oder eine gege- benenfalls halogenierte Alkyl-, Alkoxy-, Alkylcarbonyl-, Alkoxycarbonyl- oder Alkoxycarbonyloxygruppe mit 1 bis 7 C-Atomen bedeuten.

16. Polymer erhalten durch Polymerisation einer oder mehrerer polymerisierbarer Verbindungen in Gegenwart mindestens einer Verbindung nach mindestens einem der Ansprüche 1 bis 7 oder durch Polymerisation einer Zusammensetzung nach mindestens einem der Ansprüche 10 bis 15.

17. Anisotrope Polymerfolie erhalten durch Polymerisation einer polymerisierbaren Flüssigkristallzusammensetzung nach mindestens einem der Ansprüche 10 bis 15, die in ihrer Flüssigkristallphase ausgerichtet ist.

18. Verwendung einer Verbindung, einer Zusammensetzung, eines Polymers oder einer Polymerfolie nach mindestens einem der Ansprüche 1 bis 7 und 10 bis 17 in Flüssigkristallanzeigen, optischen Folien, Polarisatoren, Kompensatoren, Strahlteilern, reflexiven Folien, Orientierungsschichten, Farbfiltern, holographischen Elementen, Heißprägefolien, farbigen Abbildungen, dekorativen oder Sicherheitsmarkierungen, Flüssigkristallpigmenten, Klebstoffen, Kunstharzen mit anisotropen mechanischen Eigenschaften, Kosmetika, Diagnostika, in der nichtlinearen Optik, optischen Datenspeicherung, als chirale Dotierstoffe, in elektronischen Vorrichtungen wie Feldeffekttransistoren (field effect transistor - FET) als Komponenten integrierter Schaltungen, als Dünnfilmtransistoren in Flachbildschirmanwendungen oder für RFID-Tags (Radio Frequency Identification - RFID), in Halbleiterkomponenten für Anwendungen mit organischen Leuchtdioden (organic light emitting diode - OLED), Elektrolumineszenzanzeigen oder Hintergrundbeleuchtungen von Flüssigkristalldisplays, für Photovoltaik- oder Sensorvorrichtungen, als Elektrodenmaterialien in Batterien, als Photoleiter, für elektrophotographische Anwendungen oder elektrophotographische Aufzeichnung.

## Revendications

1. Composé de la formule I4 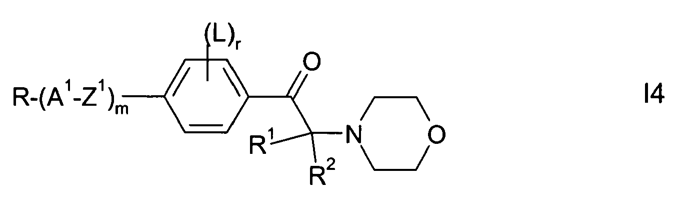 dans laquelle
R est alkyle en chaîne droite ou ramifié avec 1 à 20 atomes de C, qui peut être non substitué, mono- ou polysubstitué par F, Cl, Br, I ou CN, étant égale- ment possible pour un ou plusieurs groupes CH₂ non adjacents qu'ils soient remplacés, dans chaque cas indépendamment l'un de l'autre, par -O-, -S-, -NH-, -NR⁰-, -SiR⁰R⁰⁰-, -CO-, -COO-, -OCO-, -OCO-O-, -S-CO-, -CO-S-, -CH=CH- ou -C≡C- de telle sorte que des atomes de O et/ou S ne soient pas liés directement l'un à l'autre,
R⁰ et R⁰⁰ sont indépendamment l'un de l'autre H ou alkyle avec 1 à 4 atomes de C,
R¹ et R² sont méthyle ou éthyle, dans lequel -CR¹R²- est diffé- rent de -C(CH₃)(C₂H₅)-
A¹ est 1,4-phénylène, qui est non substitué, ou mono- ou polysubstitué par L,
L est F,
r est 0,1, 2, 3 ou 4,
Z¹ est -C≡C- ou une liaison simple, et
m est 2, 3 ou 4.

2. Composé selon la revendication 1, dans lequel le groupe -(A¹-Z¹)ₘ- est choisi parmi la liste suivante de groupes préférés, qui comprend également leurs images miroir, dans lequel Phe dans ces groupes est 1,4-phénylène, Phe L est un groupe 1,4-phénylène qui est substitué par 1 à 4 groupes L comme définis dans la revendication 1
- Phe-Z-Phe-Z- II-3
- PheL-Z-Phe-Z- II-6
- PheL-Z-PheL-Z- II-8
- Phe-Z-Phe-Z-Phe-Z- II-9
- Phe-Z-Phe-Z-PheL-Z- II-15
- Phe-Z-PheL-Z-Phe-Z- II-16
- PheL-Z-Phe-Z-Phe-Z- II-17
- PheL-Z-Phe-Z-PheL-Z- II-18
- PheL-Z-PheL-Z-Phe-Z- II-19
- PheL-Z-PheL-Z-PheL-Z- II-20
et dans lequel Z dans chaque cas indépendamment a l'une des significations de Z¹ comme donnée dans la revendication 1.

3. Composé selon la revendication 1 ou 2, dans lequel R est un radical alkyle ou alcoxy qui est en chaîne droite et qui est éthyle, propyle, butyle, pentyle, hexyle, heptyle, octyle, éthoxy, propoxy, butoxy, pentoxy, hexoxy, heptoxy, ou octoxy, en outre méthyle, nonyle, décyle, undécyle, dodécyle, tridécyle, tétradécyle, pentadécyle, nonoxy, décoxy, undécoxy, dodécoxy, tridécoxy ou tétradécoxy.

4. Composé selon au moins l'une des revendications 1 à 3, dans lequel R est un groupe alkyle ou alcoxy qui est au moins mono-substitué par halogène, est en chaîne droite, et halogène est F ou Cl.

5. Composé selon la revendication 4, dans lequel R est multi-substitué par F.

6. Composé selon au moins l'une des revendications 1 à 5, choisi parmi les formules suivantes 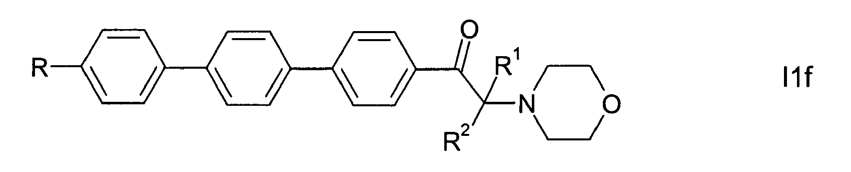 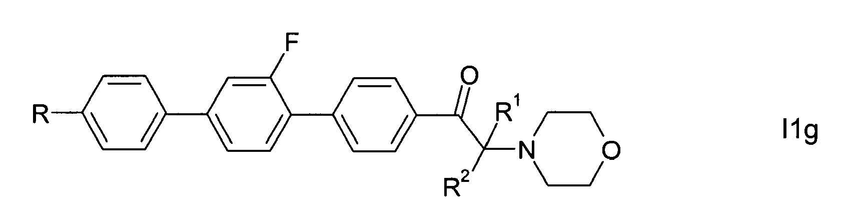 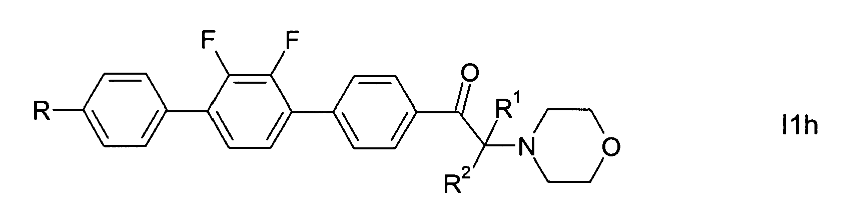 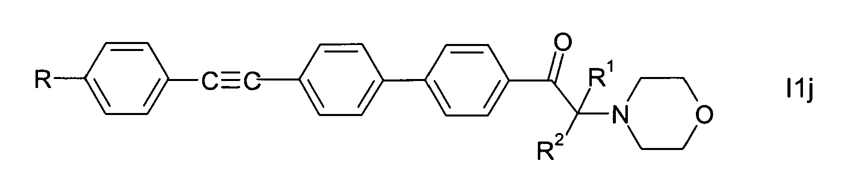 dans lesquelles R et R¹⁻² ont les significations données dans les revendications 1 à 6.

7. Composé selon la revendication 6, dans lequel R est alkyle ou alcoxy avec 1 à 8 atomes de C.

8. Utilisation d'un composé selon au moins l'une des revendications 1 à 7 comme photoïnitiateur.

9. Utilisation selon la revendication 8 pour la préparation de polymères ou de films polymère par polymérisation ou copolymérisation d'une composition de cristal liquide polymérisable comprenant au moins un composé de la formule I4 selon au moins l'une des revendications 1 à 7, ou par polymérisation d'un ou plusieurs composés polymérisables en présence d'au moins un initiateur de la formule I4 selon au moins l'une des revendications 1 à 7.

10. Composition polymérisable comprenant au moins un composé selon au moins l'une des revendications 1 à 7 et au moins un composé polymérisable.

11. Composition polymérisable selon la revendication 10, **caractérisée en ce qu'**elle est une composition de cristal liquide.

12. Composition polymérisable selon la revendication 10 ou 11, **caractérisée en ce qu'**elle comprend 0,01 à 10% d'un initiateur de la formule I4 selon une ou plusieurs des revendications 1 à 7.

13. Composition polymérisable selon au moins l'une des revendications 10 à 12, **caractérisée en ce que**, en addition à l'initiateur de la formule I4, elle comprend d'autres initiateurs.

14. Composition polymérisable selon au moins une des revendications 10 à 13, **caractérisée en ce qu'**elle comprend au moins un composé mésogène polymérisable ayant un groupe fonctionnel polymérisable et au moins un composé mésogène polymérisable ayant deux groupes fonctionnels polymérisables ou plus.

15. Composition polymérisable selon au moins une des revendications 10 à 14, **caractérisée en ce que** les composés polymérisables sont choisis parmi les formules suivantes 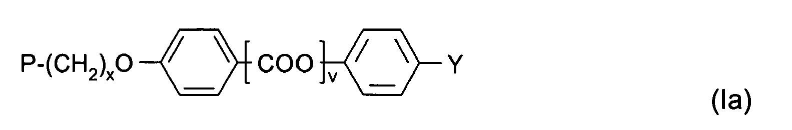 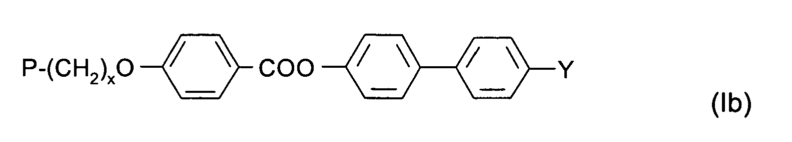 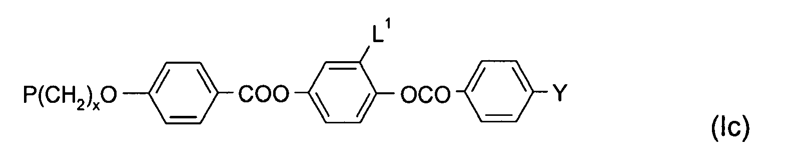 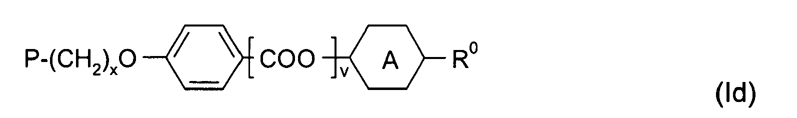 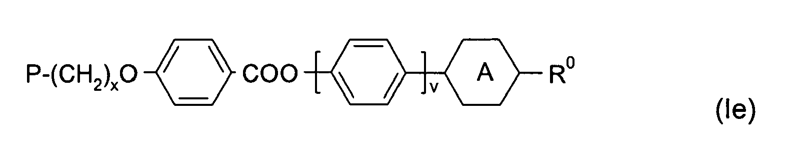 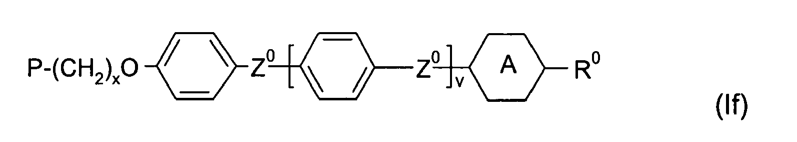 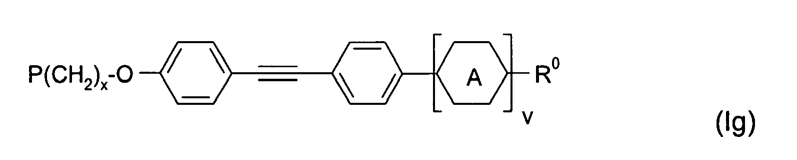 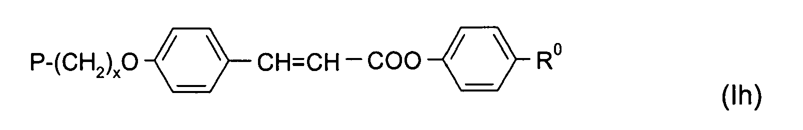 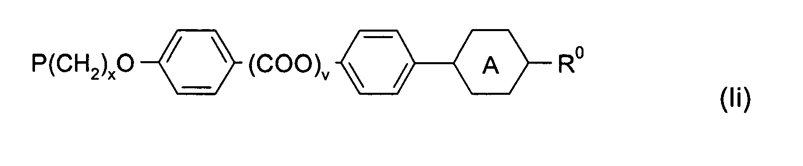 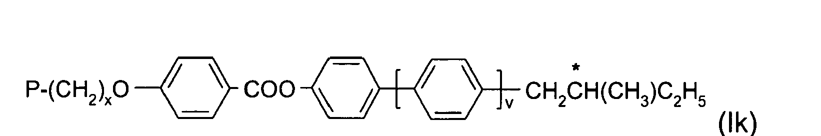 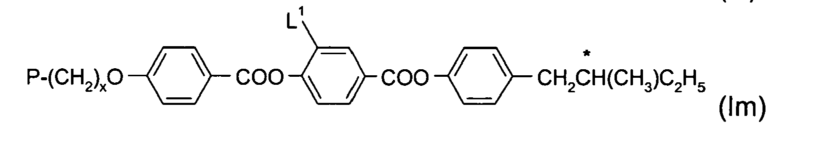 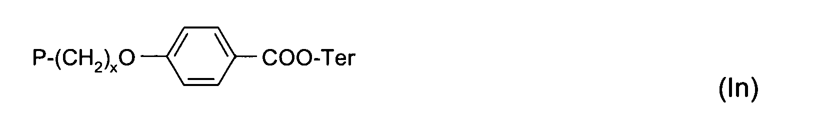 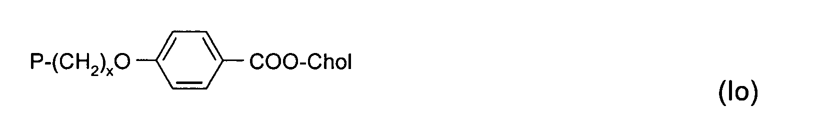 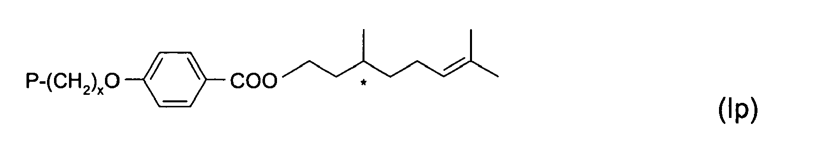 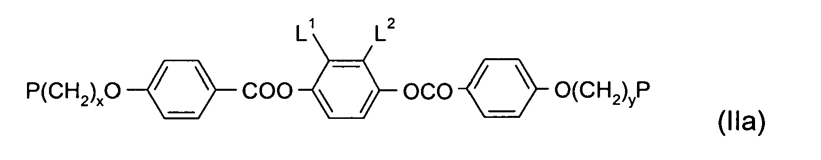 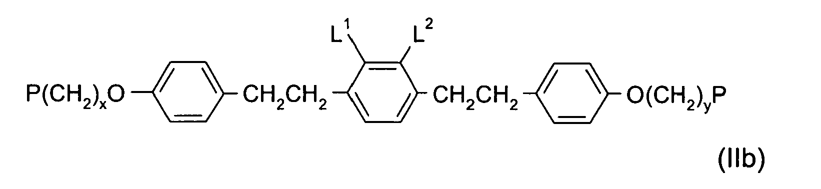 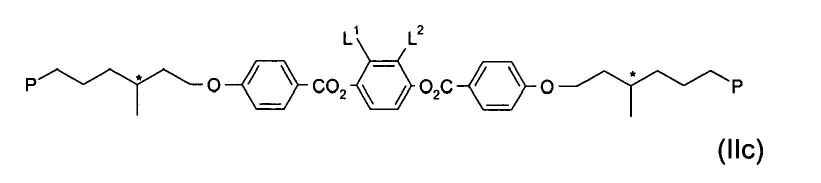 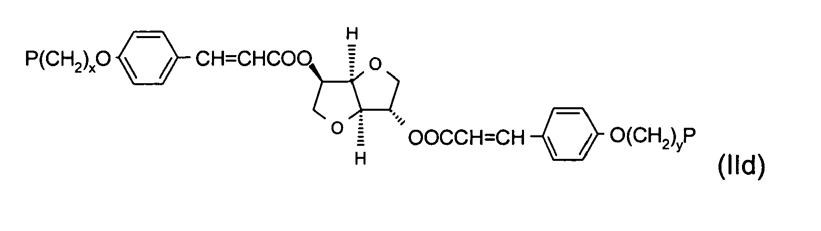 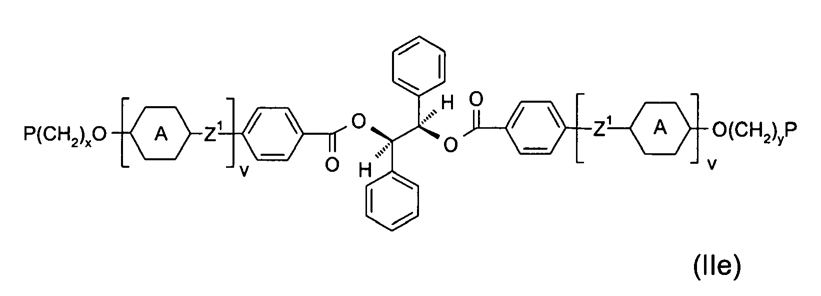 dans lesquelles
P est un groupe acryle, méthacryle, vinyle, vinyloxy, éther de propényle, époxy ou styryle,
x et y sont chacun indépendamment 1 à 12,
A est 1,4-phénylène qui est optionnellement mono- di ou tri- substitué par L1 ou 1,4-cyclohexylène,
v est 0 ou 1,
Z⁰ est -COO-, -OCO-, -CH₂CH₂- ou une liaison simple,
Y est un groupe choisi parmi F, Cl, CN, NO₂, OH, OCH₃, OCN, SCN, un groupe carbonyle ou carboxyle optionnel- lement fluoré avec jusqu'à 4 atomes de C ou un groupe alkyle ou alcoxy mono- oligo- ou polyfluoré avec 1 à 4 atomes de C,
Ter est un radical terpénoïde,
Chol est un groupe cholestéryle,
R⁰ est un groupe alkyle avec 1 à 12 atomes de C ou un groupe alcoxy avec 2 à 12 atomes de C, et
L¹ et L² sont chacun indépendamment H, F, Cl, CN ou un groupe alkyle, alcoxy, alkylcarbonyle, alcoxycarbonyle ou alcoxycarbonyloxy optionnellement halogéné avec 1 à 7 atomes de C.

16. Polymère obtenu par polymérisation d'un ou plusieurs composés polymérisables en présence d'au moins un composé selon au moins une des revendications 1 à 7 ou par polymérisation d'une composition selon au moins une des revendications 10 à 15.

17. Film polymère anisotrope obtenu par polymérisation d'une composition de cristal liquide polymérisable selon au moins une des revendications 10 à 15 qui est orientée dans sa phase cristal liquide.

18. Utilisation d'un composé, d'une composition, d'un polymère ou d'un film polymère selon au moins une des revendications 1 à 7 et 10 à 17 dans des affichages à cristaux liquides, des films optiques, des polariseurs, des compensateurs, des séparateurs de faisceau, des films réfléchissants, des couches d'alignement, des filtres couleur, des éléments holographiques, des feuilles de matriçage à chaud, des images en couleur, des marquages décoratifs ou de sécurité, des pigments de cristal liquide, des adhésifs, des résines synthétiques avec des propriétés mécaniques anisotropes, des cosmétiques, des diagnostics, des optiques non linéaires, un stockage d'information optique, comme dopants chiraux, dans des dispositifs électroniques tels que des transistors à effet de champ (FET) comme composants de circuit intégré, comme transistors à film mince dans des applications d'affichage à écran plat ou pour des étiquettes d'Identification Radio Fréquence (RFID), dans des composants de semiconduction pour des applications de diodes émettrices de lumière organiques (OLED), des affichages ou éclairages arrière électroluminescents d'affichages à cristaux liquides, pour des dispositifs photovoltaïques ou de capteur, comme matériaux d'électrode dans des batteries, comme photoconducteurs, pour des applications électrophotographiques ou un enregistrement électrophotographique.
